# EUROPEAN PATENT SPECIFICATION

(11) **EP 2 270 133 B1**
(45) Date of publication and mention of the grant of the patent: **24.06.2015**
(21) Application number: 09735168.8
(22) Date of filing: 22.04.2009
(51) Int. Cl.: C12N 1/20, A23C 9/123, A61K 35/74, C12R 1/01, C12N 1/36

(54) **METHOD FOR OBTAINING A NOVEL STRAIN OF BIFIDOBACTERIUM BIFIDUM WITH ACTIVITY AGAINST INFECTION BY HELICOBACTER PYLORI**
VERFAHREN ZUR GEWINNUNG EINES NEUEN STRANGS AUS BIFIDOBACTERIUM BIFIDUM MIT WIRKUNG GEGEN INFEKTIONEN DURCH HELICOBACTER PYLORI
OBTENTION D'UNE NOUVELLE SOUCHE DE BIFIDOBACTERIUM BIFIDUM ACTIVE CONTRE L'INFECTION PAR HELICOBACTER PYLORI

(30) Priority: 22.04.2008 ES 200801154
(43) Date of publication of application: 05.01.2011
(73) Proprietor: Corporación Alimentaria Peñasanta (Capsa), Asturias (ES)
(72) Inventor: ECHEVARRIA, Francisco, Javier, E-33199 Granada - Siero Asturias (ES); IGLESIAS BARCIA, José, Ramón, E-33199 Granada - Siero Asturias (ES); BALBARIE, Philippe, E-33199 Granada - Siero Asturias (ES); CASINOS RAMO, Beatriz, E-33199 Granada - Siero Asturias (ES); CHENOLL CUADROS, Empar, E-33199 Granada - Siero Asturias (ES); ENRIQUE LÓPEZ, María, E-33199 Granada - Siero Asturias (ES); BATALLER LEIVA, Esther, E-33199 Granada - Siero Asturias (ES); GENOVÉS MARTÍNEZ, Salvador, E-33199 Granada - Siero Asturias (ES); RAMÓN VIDAL, Daniel, E-33199 Granada - Siero Asturias (ES)
(74) Representative: Carlos Hernando, Borja
(86) International application number: PCT/ES2009/000219
(87) International publication number: WO 2009/130349

(56) References cited:
- EP-A1- 1 911 457
- WO-A1-97/24371
- WO-A2-00/74712
- WO-A2-2007/096855
- WO-A2-2007/096855
- CA-A1- 2 616 054
- JP-A- 6 128 287
- JP-A- 6 128 287
- FRANCESCHI FRANCESCO ET AL: "Role of probiotics in patients with Helicobacter pylori infection", HELICOBACTER, BLACKWELL SCIENCE, MALDEN, MA, US, vol. 12, no. Suppl. 2, 1 November 2007 (2007-11-01), pages 59-63, XP002571863, ISSN: 1083-4389, DOI: 10.1111/J.1523-5378.2007.00565.X
- CHEIKHYOUSSEF A ET AL: "Antimicrobial proteinaceous compounds obtained from bifidobacteria: From production to their application", INTERNATIONAL JOURNAL OF FOOD MICROBIOLOGY, ELSEVIER SCIENCE PUBLISHERS, AMSTERDAM, NL, vol. 125, no. 3, 31 July 2008 (2008-07-31) , pages 215-222, XP022941428, ISSN: 0168-1605, DOI: 10.1016/J.IJFOODMICRO.2008.03.012 [retrieved on 2008-04-01]
- E. CHENOLL ET AL: "Novel Probiotic Bifidobacterium bifidum CECT 7366 Strain Active against the Pathogenic Bacterium Helicobacter pylori", APPLIED AND ENVIRONMENTAL MICROBIOLOGY, vol. 77, no. 4, 15 February 2011 (2011-02-15), pages 1335-1343, XP55038791, ISSN: 0099-2240, DOI: 10.1128/AEM.01820-10
- COLLADO M.C ET AL.: 'Antimicrobial peptides are among the antagonistic metabolites produced by Bifidobacterium against Helicobacter pylori.' INTERNATIONAL JOURNAL OF ANTIMICROBIAL AGENTS. vol. 25, no. 5, January 2005, pages 385 - 391, XP008133521
- MIKI,K. ET AL.: 'Effect of Bifidobacterium bifidum Fermented Milk on Helicobacter pylori and Serum Pepsinogen Levels in Humans.' JOURNAL OF DAIRY SCIENCE. vol. 90, no. 6, pages 2630 - 2640, XP026955932
- VALENTINA TAVERNITI ET AL: "The immunomodulatory properties of probiotic microorganisms beyond their viability (ghost probiotics: proposal of paraprobiotic concept)", GENES & NUTRITION ; STUDYING THE RELATIONSHIP BETWEEN GENETICS AND NUTRITION IN THE IMPROVEMENT OF HUMAN HEALTH, SPRINGER-VERLAG, BERLIN/HEIDELBERG, vol. 6, no. 3, 16 April 2011 (2011-04-16), pages 261-274, XP019931658, ISSN: 1865-3499, DOI: 10.1007/S12263-011-0218-X
- LUBITZ PETRA ET AL: "Applications of Bacterial Ghosts in Biomedicine", ADVANCES IN EXPERIMENTAL MEDICINE AND BIOLOGY; PHARMACEUTICAL BIOTECHNOLOGY, SPRINGER, US, vol. 655, 1 January 2009 (2009-01-01), pages 159-170, XP008145631, ISSN: 0065-2598, DOI: 10.1007/978-1-4419-1132-2_12 [retrieved on 2010-01-04]
- ADAMS C A: "The probiotic paradox: Live and dead cells are biological response modifiers", NUTRITION RESEARCH REVIEWS, CAMBRIDGE UNIVERSITY PRESS, CAMBRIDGE, GB, vol. 23, no. 1, 1 January 2010 (2010-01-01), pages 37-46, XP009145190, ISSN: 0954-4224, DOI: 10.1017/S0954422410000090

### SEQUENCE LISTING

<110> Corporacion Alimentaria Penasanta
<120> Obtenei6n de una nueva cepa de Bifidobacterium bifidum con actividad frente a la infección por Helicobacter pylori
<130> AX090058WO
<150> P200801154 <151> 2008-04-22
<160> 19
<170> Patent In version 3.3
<210> 1
   <211> 10
   <212> PRT
   <213> Artificial
<220>
   <223> peptido procedente de la caseina bovina 1139.57 Da
<400> 1
   Val Tyr Pro Phe Pro Gly Pro Ile His Asn
   1               5                   10
<210> 2
   <211> 16
   <212> PRT
   <213> Artificial
<220>
   <223> péptido procedente de la caseína bovina de 1783.95 Da
<400> 2
   Pro Pro Phe Leu Gln Pro Glu Val Met Gly Val Ser Lys Val Lys Glu
   1               5                   10                  15
<210> 3
   <211> 16
   <212> PRT
   <213> Artificial
<220>
   <223> péptido procedente de la caseína bovina de 1889.97 Da
<400> 3
   Asp Lys Ile His Pro Phe Ala Gln Thr Gln Ser Leu Val Tyr Pro Phe
   1               5                   10                  15
<210> 4
   <211> 16
   <212> PRT
   <213> Artificial
<220>
   <223> péptido procedente de la caseína bovina de 1978.99 Da
<400> 4
   Pro Tyr Pro Gln Arg Asp Met Pro Ile Gln Ala Phe Leu Leu Tyr Gln
   1               5                   10                  15
<210> 5
   <211>. 17
   <212> PRT
   <213> Artificial
<220>
   <223> péptido procedente de la caseína bovina.
<400> 5
<210> 6
   <211> 21
   <212> PRT
   <213> Artificial
<220>
   <223> péptido procedente de la caseína bovina 2310.26 Da
<400> 6
   Pro Gln Asn Ile Pro Pro Leu Thr Gln Thr Pro Val Val Val Pro Pro
   1               5                   10                  15
   Phe Leu Gln Pro Glu
               20
<210> 7
   <211> 577
   <212> DNA
   <213> Artificial
<220>
   <223> Secuencia de nucleótidos obtenida a partir del amplificado de DNAr 16S obtenido de la cepa CP5
<400> 7
   tcatgcgacg gagctgaaga cgacatgcac accctgtgaa cctgccccgg aagggaaacg      60
   cctatctctg gcgtcgtcgg gaacattgtc aagcccaggg taaggttctt cgcgatgcat     120
   cgaattaatc cgcatgctcc gccgcattgt gcgggccccc gtcaatttct ttgagtttta     180
   gccttgcgga cgtactcccc aggtcgggac gcttaacgcg ttagctccga cacggaacac     240
   gtggaacgtg tcccacatcc agcgctccac cgtttacgga gatggactac cagggtatct     300
   aatcctgttc gctccccacg ctatcgctcc tcagcgtcag cgacggccca gagacctgcc     360
   ttcgccatcg gagttcttcc cgatatctac acattccacc gtatacaccg ggtaattcca     420
   gtctctccta ccgctcttcc tgtcacgctc cgatacccgg cgcaaaccct ccgtctaagc     480
   gatggacttt cacaccagta cgcgacgagc cgcctacgta gccctttaac gccccaatca     540
   gaatcccgat taacgttttg ctgcgcctac agaatta                              577
<210> 8
   <211> 21
   <212> DNA
   <213> Artificial
<220>
   <223> cebador Lm26
<400> 8
   gattctggct caggatgaac g 21
<210> 9
   <211> 22
   <212> DNA
   <213> Artificial
<220>
   <223> Cebador Lm3
<400> 9
   cgggtgcgtg cccactttca tg 22
<210> 10
   <211> 21
   <212> DNA
   <213> Artificial
<220>
   <223> Cebador BiBif-1
<400> 10
   ccacatgatc gcatgtgatt g 21
<210> 11
   <211> 19
   <212> DNA
   <213> Artificial
<220>
   <223> Cebador BiBif-2
<400> 11
   ccgaaggctt gctcccaaa 19
<210> 12
   <211> 20
   <212> DNA
   <213> Artificial
<220>
   <223> Cebador BiLON-1
<400> 12
   ttccagttga tcgcatggtc 20
<210> 13
   <211> 20
   <212> DNA
   <213> Artificial
<220>
   <223> Cebador BiLON-2
<400> 13
   gggaagccgt atctctacga 20
<210> 14
   <211> 17
   <212> DNA
   <213> Artificial
<220>
   <223> Cebador BiCAT-1
<400> 14
   cggatgctcc gactcct 17
<210> 15
   <211> 18
   <212> DNA
   <213> Artificial
<220>
   <223> Cebador BiCAT-2
<400> 15
   cgaaggcttg ctcccgat 18
<210> 16
   <211> 10
   <212> DNA
   <213> Artificial
<220>
   <223> Cebador BIF
<400> 16
   agtcagccac 10
<210> 17
   <211> 10
   <212> DNA
   <213> Artificial
<220>
   <223> Cebador RFL-2
<400> 17
   cggcccctgt 10
<210> 18
   <211> 20
   <212> DNA
   <213> Artificial
<220>
   <223> Cebador 616V
<400> 18
   agagtttgat ymtggctcag 20
<210> 19
   <211> 15
   <212> DNA
   <213> Artificial
<220>
   <223> Cebador 699R
<400> 19
   rgggttgcgc tcgtt 15

## Description

### Field of the Art

The present invention relates to a method for obtaining a novel strain of the species *Bifidobacterium bifidum* which is capable of inhibiting the growth of *Helicobacter pylori* (*H. pylori*) by a molecular mechanism present in this strain and absent in other strains of the same genus. The use of this microorganism can be applied in the agri-food and pharmaceutical sectors.

### State of the Art

The first description of the pathogenic bacterium *Helicobacter pylori* was given by Giulio Bizzozero¹ in 1892 upon detecting the presence of so-called spiral germs in the gastric mucosa of dogs. It was necessary to wait for almost ninety years for Robin Warren and Barry Marshall^{2,3} to achieve culturing this pathogenic bacterium for the first time in 1983 and to relate its presence to gastritis and peptic ulcer, although its presence has not been detected in the pancreatic juices of patients affected by chronic pancreatitis (Gasbarrini *et al.,* 2000)⁴. Said discovery was received with skepticism in medical circles, but two decades later, Warren and Marshall conclusively proved this relationship, obtaining the Nobel prize in medicine in 2005 for their work (Gisbert *et al*., 2004)⁵. Since then *H. pylori* has become one of the microorganisms whose name is recognized by a large part of the population. The reason for this fame is due to the high prevalence of the infection by said pathogen. It is considered that 50% of the world population is an *H. pylori* carrier. In developed countries this figure is between 15 and 40% of the population but in developing countries it increases up to 80%, inovlving 80% of the children under ten years of age (Taylor and Blazer, 1991)⁶.

From the clinical point of view, *H*. pylori is important for three reasons (Gisbert *et al*., 2004)⁵. Its infection is the most common cause of peptic ulcer, such that it is estimated that 20% of the individuals infected with this pathogen developed an ulcer at some time in their lives (Peterson, 1991⁷; Solnick and Tompkins, 1992⁸). Furthermore, in 1994, the World Health Organization and the International Agency for Research on Cancer established that there was sufficient epidemiological and histological evidence to conclude that *H. pylori* is the main risk factor for the onset of gastric cancer (Eurogast Study Group, 1993⁹; Chong *et al.,* 1994¹⁰). Subsequent studies have reaffirmed this statement to the point where it is suggested that the increased risk of gastric cancer involved in *H. pylori* infection is comparable to the one entailed by tobacco consumption in lung neoplasia. Finally, it is considered that *H*. *pylori* is responsible for an uncommon type of lymphoma, the so-called gastric MALT-type lymphoma, since it causes the chronic inflammation of lymphoid tissue which gives rise to the development of the disease (Vilella *et al.,* 2003)¹¹. Therefore, they are many authors who recommend eradicating *H. pylori* infection whenever it is detected (Fox and Wang, 2001)¹².

There is no current treatment against *H*. *pylori* infection which assures a complete cure rate (Gasbarrini *et al*., 2000)⁴. Therefore, reference is made to a "therapeutic strategy" for combating the pathogen. The most used one is the so-called triple therapy which consists of giving a treatment between 7 and 10 days with amoxicillin, clarithromycin and proton pump inhibitors having an efficacy of about 70-80% (Lind *et al.,* 1996¹³; The *Helicobacter pylori* Study Group, 1997¹⁴). The main causes of failure in the treatment are the lack of interest of the patient (it is necessary to take many pills a day and there are unpleasant side effects), inappropriate prescription and the presence of strains of *H*. *pylori* resistant to the antibiotics used (Deltenre *et al*., 1998)¹⁵. When it fails it is necessary to resort to a treatment referred to as "quadruple regimen" which combines a proton pump inhibitor, metronidazole, bismuth subcitrate and tetracycline for 7 to 14 days (Graham *et al*., 1991)¹⁶, Even so there are still failures, so the existence of novel therapies or, especially, of preventive mechanisms for *H. pylori* infection would be desirable.

In 1989, Bhatia *et al*. demonstrated in experiments with co-cultures that a commercial isolate of *Lactobacillus acidophilus* was capable of competing and inhibiting the *in vitro* growth of *H. pylori* (Bhatia *et al.,* 1989)¹⁷. It has subsequently been possible to observe similar inhibitions, although by using cell-free culture supernatants of a specific strain which were added to a culture of *H. pylori.* This approach has been successful with *Lactobacillus acidophilus* (Mrda *et al*. 1998¹⁸, Gasbarrini *et al.* 2004¹⁹), specifically with the strains referred to as CRL639 (Lorca *et al*., 2001)²⁰, LB (Coconnier *et an,* 1998)²¹, La1 (Michetti *et as,* 1999)²² and *Shirota* (Sgouras *et al*., 2004)²³, and also with *Lactobacillus delbrueckii* subsp. *bulgaricus* (Mrda *et al*. 1998)¹⁸ and with mixtures of the strains *L. acidophilus* R0052 and *Lactobacillus rhamnosus* R0011 (Johnson-Henry *et al*., 2004)²⁴. In all the cases there is a very considerable strain specificity (Hamilton-Miller, 2003)²⁵. An activity inhibiting the growth of *H*. *pylori* with cell-free supernatants of *Bacillus subtilis* (Pinchuk *et al.,* 2001)²⁶ and *Weissella confusa* (Nam *et al.,* 2002)²⁷, and with that of several bacteria typical of the oral cavity such as *Fusobacterium nucleatum, Porphyromonas gingivalis, Prevotelia inter media, Prevotella nigrescens, Prevotella oris, Streptococcus mitis, Streptococcus mutans, Streptococcus oralis, Streptococcus salivarus* and *Streptococcus sobrinus* has also been detected (Ishihara *et al*., 1997)²⁶. The latter result could explain the lack of efficacy of *H. pylori* in the colonization of the oral cavity.

There seem to be several reasons for which either these bacteria or a metabolite secreted by them into the culture medium inhibit the growth of *H. pylori.* Given that many of the selected strains are lactic acid bacteria, the possible bactericidal effect of lactic acid or of any other organic acid on *H. pylori* has been suggested (Bathia *et al*., 1989¹⁷; Aiba *et al.,* 1998²⁹; Hamilton-Miller, 2003²⁵). However, in most cases it has been specified that the lytic effect is due to a metabolite secreted into the culture medium other than lactic acid, the existence of antibiotics (Pinchuk *et al.*, 2001)²⁶, autolysins (Lorca *et al.,* 2001)²⁰ or bacteriocins (Nam *et al*., 2002)²⁷ occasionally being suggested.

In experimentation with *in vitro* cell cultures, it has been possible to observe the competition for the binding to murine gastric epithelium cells or human gastric carcinoma cells between *H. pylori* and *L. acidophilus, L. brevis* or *L*. *salivarus* strain WB1004 (Kabir *et al,* 1997)³⁰. As a consequence, by means of using these strains of lactic acid bacteria the secretion of interleukin-8 is reduced (Kabir *et al*., 1997)³⁰. A similar effect of reducing the production of interleukin-8 using the strain *Lactobacillus gasseri* OLL2716 has been demonstrated (Ushiyama ef *al.,* 2003)³¹. It has even been suggested that the competition for glycolipid receptors between *H. pylori* and *Lactobacillus reuteri* could be a strategy from which to use the latter bacterium as an effective probiotic against *H. pylori* (Mukai *et al.,* 2002)³².

All these *in vitro* results have been endorsed with *in vivo* preclinical experimentation with animal models, especially using germ-free (GF) mice or specific pathogen-free (SPF) mice. Kabir *et al.* demonstrated in 1997 that when GM mice were fed with the strain *L*. *salivarus* WB1004, they were protected against a subsequent *H*. *pylori* infection (Kabir *et al*., 1997)³⁰. Upon performing the opposite experiment, i.e., first causing the *H. pylori* infection and subsequently feeding the mice with the strain *L*. *salivarus* WB1004, the pathogen counts decreased to 1% in comparison with the animals which were not fed with the lactic acid bacterium (Kabir *et al*., 1997)³⁰. These experiments have been repeated in other laboratories (Aiba *et al*., 1998)²⁹. There are also similar results using against *H. pylori the* strain *L. gasseri* OLL2716 (Ushiyama *et al.,* 2003)³¹ and using a mixture of the prebiotic strains *L. acidophilus* R0052 and *Lactobacillus rhamnosus* R0011 (Johnson-Henry *et al*., 2004)²⁴. Recently, Sgouras *et al*. have demonstrated the inhibition of *H*. *pylori* by *L. casei* strain *Shirota* in similar experiments but using SPF mice (Sgouras *et al*., 2004)²³. There is only one study to date using conventional mice, which was successfully carried out using the strain *L. acidophilus* LB, although the experimental animals were infected with *Helicobacterfelis* instead of with *H. pylori* (Coconnier *et al*., 1998)²¹.

In relation to the clinical research with human volunteers, the two experimental models used to date should be differentiated. The first of them consists of combining the probiotic treatment with the administration of antibiotics effective against *H*. *pylori* (for a review consult Hamilton-Miller, 2003)²⁵. In the second, the bacterium with the possible probiotic effect is ingested in an isolated manner, either as a lyophilisate or in a dairy product. With respect to the combined treatment, seven trials have been carried out to date, four of them with asymptomatic subjects using *Lactobacillus* GG or *L. johnsonii* strain La1 (Armuzzi *et al.,* 2001a³³ and b³⁴ Felley *et al.,* 2001³⁵; Cremonini *et al.,* 200136) and the rest with dyspeptic patients using *L. acidophilus* (Canducci *et al*., 2000³⁷; De Francesco *et al.,* 2000³⁶; Sheu *et a*/*.,* 2002³⁹). The difficulty of these trials is determining to what degree the detected effect is due to the probiotic or to the pharmacological treatment. Even so, a positive effect of the addition of the probiotic is concluded in six of the seven assays (Canducci *et al.,* 2000³⁷; Armuzzi *et al*., 2001a³³ and b³⁴; Felley *et al.,* 2001³⁵; Cremonini *et al.,* 2002³⁶; Sheu *et al.,* 2002³⁹). In the case of using only the probiotic, eight clinical trials have been carried out. In one trial, culture supernatants of *L. johnsonii* were ingested (Michetti *et al.,* 1999²²). In the rest, different dairy products which contained the strain object of study were formulated (Mrda *et al.,* 1993⁴⁰; Scholz-Ahrens & Schrezenmeir, 2000⁴¹; Nakaji *et al.,* 2001⁴²; Wendakoon *et al*., 2002⁴³). Only three of these trials were randomized and placebo-controlled, which makes it difficult to analyze the results. Only in one of the eight clinical trials was a positive effect not detected (Wendakoon *et al.,* 2002⁴³). In the rest, significant improvements were obtained in the general condition of the individuals to a greater or lesser extent.

In the field of the invention there are various documents using antibiotics to eradicate *H. pylori* in the case of intestinal disorders, such as patent EP 0787494 A1, for example, which describes the use of rifamycin derivatives for the manufacture of a medicament for the treatment of diseases caused by *H*. *pylori* infections. Likewise, document EP 0866793 A1 describes nitroimidazole antibacterial compounds and the use thereof against *H*. *pylori* and *Mycobacterium tuberculosis.*

Patent document EP 1092040 A1 relates to a method for selecting molecules capable of inhibiting the survival of *Helicobacter,* particularly of *H. pylori, in vivo* by specifically inhibiting the urease activity encoded in the Urel gene, and to the use of these molecules to treat or prevent *H. pylori* infection.

Some documents use probiotics to eradicate *H. pylori* in case of gastrointestinal disorders. Document JP 2002322086 relates to an active agent, specifically lactoferrin, which is used as a prophylactic or therapeutic agent against *H. pylori* infection. To that end, it is included in beverages or foods in order to treat *H. pylori* infection. In addition to lactoferrin, said prophylactic or therapeutic agent against *H. pylori* infection comprises at least one microorganism belonging to the genus *Bifidobacterium.*

Patent document US 6329002 B1 relates to a food for inhibiting infection and treating gastritis, gastric and duodenal ulcers, which is effective against *H. pylori* not only *in vitro* but also *in vivo* and which comprises an effective amount of a microorganism selected from the group consisting of live strains of *Lactococcus* sp. HY49, *Lactobacillus casei* HY 2782 and *Bifidobacterium longum* BY 8001. The properties of said bacteria are boosted by the addition of egg yolk containing antibodies specific to *H. pylori.* Likewise, in patent document JP 2001258549 the probiotics *L. acidophilus* HY2177 and *L. casei* 274, having activity against *H. pylori* K551, obtained from the stomach ulcer of a patient, have been selected to make preparations against *H. pylori.*

Patent document KR 20020061040 describes lactic products for inhibiting the growth of *H, pylori* containing *L*. *gasseri, L. acidophilus* M and *Bifidobacterium lactis* Bb-12, or their culture products. Said products are used to prevent and treat gastritis and duodenal ulcers, among others, minimizing the side effects of the antibiotics which are used in the treatment against *H. pylori.*

In patent document WO 03/051132 A1, the invention relates to a method for preparing fermented food having suppressive effect against *H. pylori* and/or food-poisoning bacillus. Said method comprises the steps of: a) inoculating lactic ferments into the food, wherein said lactic ferments is selected from the group consisting of the genus *Lactobacillus,* the genus *Bifidobacterium,* preferably the species *B.infantis,* the genus *Streptococcus* and mixtures thereof, said food being selected from the group consisting of grains, fruits, vegetables and mixtures thereof; and b) incubating said inoculated mixtures at 15-35°C for 12-48 hours.

Patent document WO 00/74712 A2 relates to a dietetic, nutritional or pharmaceutical composition comprising a sufficient amount of alkaline sphingomyelinase to have a dietetic, nutritional or therapeutic effect on an individual in need of it, such as to prevent or treat, for example, different pathological conditions including carcinogenic processes, inflammation of the intestine, hypercholesterolemia and *H. pylori* infections. Sphingomyelinase can be bacterial, and the bacteria containing it are chosen from Gram-positive bacteria, Gram-negative bacteria and lactic acid bacteria, or from mixtures thereof. Sixteen strains of the genus *Lactobacillus,* 12 of the genus *Bifidobacterium* such as the species *B. bifidum,* and 3 of the genus *Streptococcus* are mentioned among the lactic acid bacteria.

Patent document WO 2007/01097 A1 describes a strain of the species *Bifidobacterium bifidum* having the following features: (1) it produces the effect of killing *Helicobacter pylori,* and (2) it shows a survival rate of 10% or higher in the case of being stored in a fermented milk beverage or in foods in aerobic conditions at 10°C for 14 days.

However, there is still a pressing need both in developed and in developing countries for effective products and methods both in the prevention and in the treatment of *H. pylori* infections, either alone or in association with therapy using antibiotics or other active agents.

Many of the works mentioned in the preceding paragraphs describe the *in vitro* inhibition of the growth of *H. pylori,* but they do not progress into the study of the underlying molecular mechanisms of said phenomenon and of the industrial use of this property. This document describes obtaining a strain of the species *Bifidobacterium bifidum,* isolated from feces of a healthy child less than a year old, fed with breast milk. Said strain is capable of inhibiting the growth of different clinical isolates of *H. pylori.* Without ruling out that said inhibition is in part due to a phenomenon of natural competition, the results in this invention conclusively demonstrate that various peptide metabolites present in the culture medium of the strain *B*. *bifidum* object of study are responsible for said inhibition. Other strains of different species of the genus *Bifidobacterium,* and even other strains of the species *B*. *bifidum,* do not present this production phenotype of said peptide complex. Said peptides have been identified as products of the enzymatic degradation of casein. Consequently, it is possible to formulate various foods and beverages containing said microorganism and casein, such that when the microorganism grows and secretes a protease, it releases the peptide complex inhibiting *H. pylori,* or by directly adding to the food or beverage the peptide complex obtained by other industrial means.

### Description of the Invention

The present invention describes a method for obtaining agents with anti-*H*. *pylori* activity, and therefore effective in treating ulcers. During the present invention, the inventors have investigated the inhibitory effect on *H. pylori* exerted by different bacteria isolated from feces. This invention is based on using the strain of *Bifidobacterium bifidum* deposited in the *Colección Española de Cultivos Type* (Spanish type Culture Collection of Microorganisms) as CECT 7366 on 28 January 2008. The inhibitory effect has been observed with the culture supernatant of strain CP5 grown in the presence of cysteine. The strain of the invention is active against the *H. pylori* infection as is demonstrated in the *in vitro* adhesion assays. The inventors have found that the supernatant of this strain has proven anti-*H*. *pylori* activity.

Strain CP5 maintains 88% viability for at least a period of 15 days when it is added to a dairy product maintained at 5°C.

Furthermore, an object of the present invention is to provide dietetic, nutritional and/or pharmaceutical compositions which are effective in the prophylaxis of an *H*. *pylori* infection. Pharmaceutical compositions are those which are useful only because of their therapeutic or prophylactic effects and which are generally formed by only the active ingredient (in this case a lyophilisate of strain CP5 of *B*. *bifidum*) with one or more suitable carriers depending on the galenic form of the medicament. Dietetic compositions are those comprising nutritional materials and other nutritional supplements in addition to the active agent.

### Object of the Invention

The present invention describes a method for obtaining agents with anti-*H*. *pylori* activity, and therefore effective in treating ulcers. During the present invention, the inhibitory effect on *H*. *pylori* exerted by different bacteria isolated from feces has been investigated. One of them is the strain of *B*. *bifidum,* referred to as CP5, deposited in the Spanish type Culture Collection of Microorganisms (CECT) with accession number CECT 7366. The inhibitory effect has been observed with the culture supernatant of strain CP5 grown in the presence of cysteine, which has proven anti-*H*. *pylori* activity, as well as with the reverse phase fraction containing the peptides SED.ID.NO:1-6, obtained from said bacterial strain.

The present invention describes the detailed characterization of strain CP5, object of the present invention, as well as obtaining the active peptides of the supernatant of the strain after the fermentation thereof, and the anti-*H*. *pylori* activity thereof.

The present application described obtaining a strain of the species *B*. *bifidum,* isolated from the feces of a healthy child less than a year old, fed with breast milk. Said strain is capable of inhibiting the growth of different clinical isolates of *H. pylori* and of several reference strains. Without ruling out that said inhibition is in part due to a phenomenon of natural competition, the results in this invention conclusively demonstrate that various peptide metabolites present in the culture medium of the strain *B. bifidum* object of study are responsible for said inhibition. Other strains of different species of the genus *Bifidobacterium,* and even other strains of the species *B*. *bifidum,* do not present this production phenotype of said peptide complex. Said peptides have been identified as products of the enzymatic degradation of casein. Consequently, it is possible to formulate various foods and beverages containing said microorganism and casein, such that when the microorganism grows and secretes a protease, it releases the peptide complex inhibiting *H. pylori,* or by directly adding to the food or beverage the peptide complex obtained by other industrial means, i.e., the use thereof as a probiotic agent in foods.

### Brief Description of the Drawings

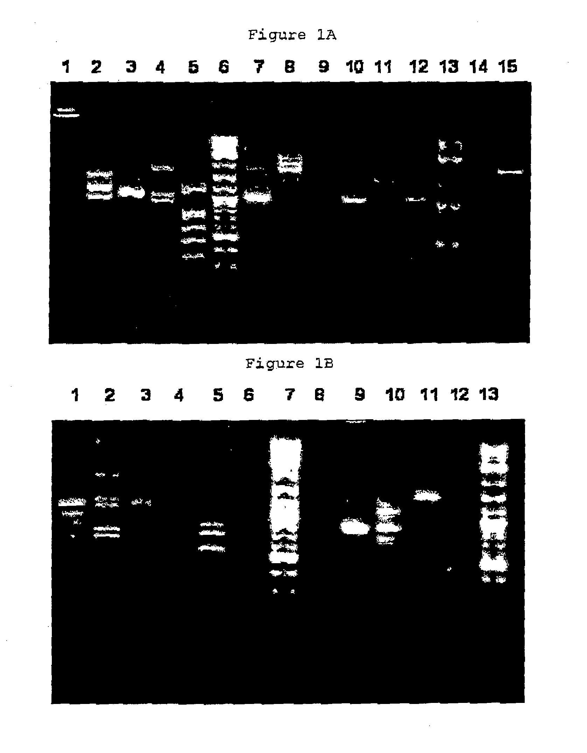

Figure 1 shows a comparison of the electrophoretic profiles obtained from the amplification of strain CP5 with primers BIF (A) and RFL-2 (B) in comparison with other strains of *Bifidobacterium* spp. and lactic acid bacteria.
In Figure 1A, the different lanes correspond to:
   *1. B. longum* CECT 7210; 2. *B. gallicum* CECT 5778; 3. *B*. *angulatum* CECT 5775; 4. *B*. *dentium* CECT 687; 5. *Lactobacillus rhamnosus* BP2; 6. 100 bp Molecular Weight Marker; 7. *Enterococcus faecalis* BP1; 8. *B. longum* CECT 4503; 9. *B*. *adolescentis* 5781; 10. *E. faecalis* BP17; 11. *B. infantis* CECT 4552; 12. *B*. *cuniculi* DSM 20435; 13. 100 bp Molecular Weight Marker; 14. *B. breve* CECT 4839; 15. *B. bifidum* CECT 7366.
In Figure 1B, the different lanes correspond to:
   *1. B. longum* CECT 7210; 2. *B. gallicum* CECT 5778; 3. *B*. *angulatum* CECT 5775; 4. *B. dentium* CECT 687; 5. *L. rhamnosus* BP2; 6. *E. faecalis* BP1; 7. 100 bp Molecular Weight Marker; 8. *B*. *longum* CECT 4503; 9. *B*. *adolescentis* 5781; 10. *E. faecalis* BP17; 11. *B. breve* CECT 4839; 12. *B*. *bifidum* CECT 7366; 13. 100 bp Molecular Weight Marker
Figure 2 shows the assay for the resistance to bile salts of the selected strains with growth at 37°C for 24 hours in anaerobiosis.
Figure 3 shows the effect of the different NaCl concentrations on the growth of the selected strains with growth at 37°C for 24 hours in anaerobic conditions.
Figure 4 shows the production of lactic acid (D/L) by strain CP-5 in culture supernatant.

### Detailed Description of the Invention

The strain of the invention is active against *H*. *pylori* infection as is demonstrated with the supernatant resulting from fermenting the strain in the *in vitro* adhesion assays. Different fractions containing at least one of the peptides SEQ.ID.NO: 1 to 6 inhibiting the growth of *H*. *pylori by* at least 70%, preferably by 80% and more preferably by 90%, have been isolated. Specifically, a fraction of said supernatant having an inhibition value of *H*. *pylori of* 94.77% probably due to the presence of all the specific peptides which have been identified as SEQ. ID. NO: 1 to 6, has been isolated.

Strain CP5 is used in the form of viable and non-viable cells. The viable cells are used by introducing the live bacteria into a non-fermented composition. The administration of such compositions thus allows the anti-*Helicobacter pylori* activity to take place in the gastrointestinal tract.

The viable and non-viable cells result from exposing strain CP5 to fermentation in a suitable medium, such as a dairy medium for example, so that the polypeptides referred to as SEQ ID.NO: 1 to 6 are produced.

Bacterial cultures obtained from strain CP5 comprising viable cells and/or non-viable cells containing at least one of the peptides SEQ.ID.NO: 1 to 6 are also part of the invention.

Providing dietetic or nutritional and/or pharmaceutical compositions which are effective in the prophylaxis of an *H. pylori* infection is also an object of the present invention. Strain CP5 can be used in the form of viable cells and acts as a probiotic. According to the definition adopted by the FAO, probiotics are "live microorganisms which when administered in adequate amounts confer a health benefit on the host" and some yeasts and bacteria are generally used as such, the most widely used being bacteria of the genus *Lactobacillus*.

Dietetic compositions are those compositions comprising in addition to the active agent other materials and nutritional supplements. The supernatant of strain CP5 has a viability of 88% for at least a period of 15 days when it is added to a dairy product maintained at 5°C. Said compositions can be mixed with other probiotics, for example in yogurts.

Pharmaceutical compositions are those which are useful only because of their therapeutic and/or prophylactic effects and which are generally formed by only the active ingredient (in this case a culture or a lyophilisate of strain CP5 of *B*. *bifidum* or peptides obtained from the supernatant of the fermentation of said strain with one or more suitable adjuvants and/or carriers depending on the galenic form of the medicament.

Depending on the type of composition, nutritional or pharmaceutical, and on the physical or galenic form thereof, the person skilled in the art knows how to choose the suitable excipients and carriers.

In said compositions, strain CP5 is present in an amount of about 10⁵ to 10¹¹ cfu/g, "cfu" being a colony-forming unit which is defined as "the number of bacterial cells which are detected per microbiological count in agar plates per gram of composition".

The characterization of strain CP5, as well as the peptides isolated from the supernatant after fermenting said strain and the anti-*H. pylori* activity thereof are described below.

The peptides of the invention can be used in the aforementioned compositions with bacteria of the invention.

The detailed characterization of the strain of the present invention, as well as obtaining the supernatant of the strain after the fermentation thereof and the anti-*H*. *pylori* activity thereof, are described below.

### Examples

### 1. Isolating microorganisms.

To isolate the strains of *Bifidobacterium,* 1 g of feces was dissolved in PBS buffer (phosphate buffered saline, pH 7.0) homogenizing the sample by means of macerating for 3 minutes in a Stomacher (Bagmixer 400P, Interscience Worlwide, France). A 1 mL aliquot of the homogenate was inoculated into Man Rogosa Sharpe (MRS) agar adjusted to pH 3.0 with HCl supplemented with 0.05% (w/v) of cysteine (MRS-C; Sigma, St. Louis, Mo.). It was incubated for 17 hours at 37°C in anaerobic conditions and different dilutions of this preincubation medium were seeded in plates with MRS-C medium and *Bifidobacterium* selective medium (BSM, Fluka, Buchs, Switzerland) and *Bifidobacterium* medium agar (BFM; Nebra, 1999¹) and they were incubated for 36 to 72 hours at 37°C in anaerobic conditions. The colony morphology and the type of cell wall of the strains obtained were determined by Gram staining. It was observed that 70% of the total strains analyzed belonged to the genus *Bifidobacterium.*

### 2. In vitro assays of activity against H. pylori of the different bifidobacteria and their culture supernatants.

The activity of the culture supernatants of six bifidobacteria (CP1, CP2, CP3, CP4, CP5 and CP6) was analyzed versus two reference strains of *H. pylori* (NCTC 11637 and NCTC 11638) and six *H. pylori* isolates of a clinical origin (A1, A2, A3, A4, A5 and A6). In a first assay, the antibacterial activity was evaluated by techniques of agar diffusion of the selected culture supernatants of the bifidobacteria versus the reference strains *H. pylori* NCTC 11637 and NCTC 11638.

Two subcultures of lactic acid bacteria were formed with a 1 % inoculum (w/v) in 3 ml of MRS medium (Pronadisa) supplemented with 0.05% (w/v) of L-cysteine (Scharlau) and incubating in anaerobic conditions for 24 hours at 37°C. The lactic acid bacteria thus grown were again inoculated in the same conditions into 50 mL of MRS-C and MRS-C supplemented with 1% (v/v) Tween 80, respectively, and incubated for 17 hours after which the culture supernatants were obtained by centrifugation. These supernatants were neutralized with 1N NaOH at pH 6.5 and sterilized by filtration through filters with a pore size of 0.22 µm (Sartorius). They were subsequently concentrated ten times by lyophilization and they were resuspended in 50 mM sodium phosphate buffer at pH 6.5. The samples were stored at -80°C until the use thereof.

Assays were performed in liquid medium for the antibacterial capacity of the supernatants of the aforementioned 6 strains of bifidobacteria versus the strain *H*. *pylori* NCTC 11637. Using the Brain Heart Infusion medium supplemented with 5% (v/v) fetal bovine serum (Invitrogen) as a base, a 1% inoculum (w/v) of the indicator strain was made in 96-well polystyrene plates from a bacterial suspension with an optical density at 600 nm equal to 2.5 ± 0.05 for the purpose of standardizing the number of microorganisms at 10⁴ cfu/mL. 25 µL of the supernatants (SBN) were added to said medium for a final assay volume of 200 µL per well. The mixture was incubated at 37°C in microaerophilic conditions for 3-4 days after which its optical density was measured at 600 nm. The assay included controls of inoculated culture medium without supernatant and of non-inoculated culture medium with supernatant. The obtained results are shown in Table 1. Using this methodology a proliferation inhibitory effect was observed by adding all the assayed supernatants to the medium.

**Table 1. Inhibition of H. pylori NCTC 11637 proliferation by the supernatants of bifidobacteria CP1, CP2, CP3, CP4, CP5 and CP6.**

| Supernatant | CP1 | CP2 | CP3 | CP4 | CP5 | CP6 |
|---|---|---|---|---|---|---|
| % Inhibition | 73.46 | 70.25 | 67.69 | 77.14 | 81.94 | 72.57 |

At the same time, inhibition assays were performed by means of agar diffusion. The neutralized supernatants (SBN) were used in determining the antimicrobial activity versus strains *H. pylori* NCTC 11637 and NCTC 11638. To that end 1.8 x 10⁸ cfu/mL (McFarland = 6) of the indicator strain of *H. pylori* were inoculated into 1 mL of saline and was extended on the surface of Trypticase Soy Agar supplemented with 5% (v/v) horse blood (Oxoid). The samples of neutralized supernatant (50 µL) were deposited in the different cells (5 mm in diameter) made in the agar. The inhibition areas were observed after 2-3 days of incubation at 37°C in microaerophilic conditions (Campygen; Oxoid). In these conditions, no inhibition effect was observed on the *H. pylori* isolate. To increase inhibition, the conditions of the bifidobacteria growth culture medium were modified in order to improve the production of the inhibitors. The bifidobacteria therefore grew in the same conditions as in the previous case but using the MRS-C medium (Oxoid) which was supplemented with 1% (w/v) Tween 80. The supernatants were treated as in the previous case and their activity was analyzed by the agar diffusion method verses the strains of *H. pylori* NCTC 11637 and NCTC 11638 (Table 2) and the six clinical isolates of *H. pylori* A1-A6 (Table 3). As a result of the study, the presence of larger inhibiting areolas versus the strains of *H. pylori* was confirmed in all the assayed culture supernatants, although strains CP3, CP4 and CP5 showed the greatest activity.

**Table 2. Plate antimicrobial activity of the culture supernatants of strains of Bifidobacterium grown for 17 hours in MRS-C medium versus control strains of H. pylori NCTC 11637 and NCTC 11638 supplemented with 1% (w/v) Tween 80 versus H. pylori isolates.**

| | Diameter of the bacterial growth inhibition area (mm) | |
|---|---|---|
| Strains *Bifidobacterium* | *H. pylori* NCTC 11637 | *H.pylori* NCTC 11638 |
| CP1 | 1 | 5.5 |
| CP2 | 7.25 | 5 |
| CP3 | 7.25 | 6 |
| CP4 | 6.25 | 7.5 |
| CP5 | 7.25 | 7 |
| CP6 | 6 | 5.25 |

**Table 3. Plate antimicrobial activity of the culture supernatants of strains of Bifidobacterium grown in MRS-C medium supplemented with 1% (w/v) Tween 80 versus H. pylori isolates.**

| | Diameter of the *H. pylori* isolate growth inhibition area (mm) | | | | | |
|---|---|---|---|---|---|---|
| Strains *Bifidobacterium* | A1 | A2 | A3 | A4 | A5 | A6 |
| CP1 | 5.2 | 6.7 | 2.5 | 5.7 | 2.7 | 4.5 |
| CP2 | 6 | 6.5 | 4.5 | 5 | 6.2 | 5.5 |
| CP3 | 6.6 | 6.3 | 5 | 5 | 5 | 4.5 |
| CP4 | 6 | 6 | 4.7 | 6 | 6 | 6 |
| CP5 | 6.7 | 7.8 | 5 | 5.8 | 6.7 | 6.5 |
| CP6 | 5 | 7.2 | 2.5 | 5 | 6 | 4 |

### 3. Inhibition assays of the adhesion of the pathogen to mucus by competition with lactic acid bacteria.

In view of the obtained results, an inhibition study of the adhesion of three strains of *H. pylori* (the clinical isolates A1 and A2 and control strain NCTC 11637) by the strains under study was carried out.

Type III porcine mucine (Sigma) was used at a protein concentration of 0.5 mg/mL. To that end, the total protein concentration was determined according to the Bradford method (Biorad) using bovine serum albumin (BSA) as a standard.

The *H. pylori* cells grown in microaerophilia for 36 hours at 37°C were washed twice with HEPES (N-2-_hydroxyethylpiperazine-N-2-ethanesulfonic acid) - Hanks buffer (10 mM HEPES in Hanks' balanced salts, pH 7.4; Sigma) after which the microorganism concentration was determined by means of measuring absorbance at 600 nm, being adjusted to an OD of 0.25 + 0.05 for the purpose of standardizing the number of microorganisms. The cells were subsequently fluorescently labeled by adding to the buffer 10 µ1/mL of 100 mM carboxyfluorescein diacetate (CFDA; Calbiochem) and they were incubated at 37°C for 45 minutes, protecting the mixture from the light.

The assay consisted of adding 100 µL of the unlabeled probiotic cells to wells of multiwell polystyrene plates (Maxisorp, Nunc) where the mucus had previously been fixed, and they were incubated for 1 hour at 37°C. For the fixing thereof, the mucus was previously dissolved in HEPES-Hanks buffer (HH) at the desired concentration and was immobilized on the Maxisorp-Nunc plates by means of adding 100 µl of this solution to each of the wells. After incubation at 4°C overnight, the non-immobilized mucus residue was withdrawn by means of two consecutive washings with 200 µl of HH buffer. After the last washing, 100 µl of HH buffer were added to each well. The lactic acid bacteria not adhered to the mucus were removed by means of two washings with 200 µL of HH buffer and 100 µL of cells of the pathogen *H. pylori* labeled with CFDA were added to the wells and incubated at 37°C for 1 hour. The pathogens not adhered to mucus were removed by means of two washings with HH buffer. The cells adhered to mucus were released by means of scraping and were lysed with 1% (w/v) SDS in 0.1 N of NaOH at 60°C for 1 hour. The released fluorescence was measured at λₑₓ=485 nm; λₑₘ=518 nm in the Fluoreskan Ascent FL analyzer. The results were expressed as the percentage of fluorescence recovered after adhesion in relation to the fluorescence of the bacterial suspension added to the wells. The percentage of inhibition of the adhesion was calculated as the difference between the adhesion of the pathogen in the absence and in the presence of bifidobacteria. The assays were performed in three independent experiments and each of them was performed in quadruplicate. The mean results are shown in Table 4.

**Table 4. Percentage of inhibition of the adhesion of three strains of H. pylori to mucus per culture together with the selected bifidobacteria.**

| | % Inhibition of the adhesion to mucus | | |
|---|---|---|---|
| Strains *Bifidobacterium* | A1 | A2 | NCTC 11637 |
| CP1 | 4.00±0.38 | 2.11±0.99 | -0.52±1.65 |
| CP2 | -3.66±0.47 | 6.72±0.23 | 4.71±1.85 |
| CP3 | -4.00±5.61 | 3.80±1.15 | 4.84±0.54 |
| CP4 | -6.83±1.62 | 1.74±1.87 | 4-97±0.76 |
| CP5 | 5.42±5.48 | 3.59±0.75 | 8.17±2.60 |
| CP6 | 3.89+4.82 | 1.27±6.07 | -7.46±0.08 |

The negative values imply an increase of the adhesion

For two of the three strains of *H*. *pylori* analyzed, the results indicated substantial inhibition of the adhesion by strain CP5.

Because of these results, together with those obtained in the *in vitro* studies of the inhibition of *H. pylori* proliferation, strain CP5 together with a control of the commercial probiotic *Lactobacillus rhamnosus* GG was ruled out for subsequent studies.

### 4. Identification and characterization of strain CP5

### 4.1 Extraction and purification of genomic DNA

The bacterial genomic DNA (gDNA) was isolated from a culture of CP5 cells in MRS-C broth for 17 hours at 37°C in anaerobic conditions. These cells were collected, washed with TE buffer (10 mM Tris-HCl and 1 mM EDTA) and resuspended in 500 µl of TE buffer to which 20 µl of a lysozyme solution (50 mg/mL) were added. This mixture was incubated at 37°C for an hour and a half. After this incubation, 30 µl of 10% SDS (w/v) and 3 µl of a proteinase K solution (10 mg/mL) were added and it was incubated for 1 hour at 37°C. The DNA suspension was cleaned by adding to the sample 100 µl of a 5 M NaCl solution and 80 µl of a solution of cetyltrimethylammonium bromide (CTAB) and NaCl (10% CTAB (w/v) and 0.7 M NaCl). This mixture, which is well dissolved, was incubated at 65°C for 10 minutes.

The DNA was purified by means of the extraction technique using a volume of phenol-chloroform-isoamyl alcohol (25:24:1). The nucleic acids were precipitated by means of adding 500 µl of isopropanol, stirring and leaving at room temperature for 10 minutes, after which time it was centrifuged at 12000 rpm for 15 minutes. The precipitated DNA was washed with 70% (v/v) cold ethanol. The clean and precipitated DNA was vacuum-dried and resuspended in 50 µl of TE buffer. 3 µl of RNAse solution (10 mg/mL) were added to each sample, incubating the mixture at 37°C for 30 minutes. The DNA concentration was determined in 0.7% agarose gel (w/v) in TAE buffer (242 g base Tris, 57.1 mL glacial acetic acid, 100 mL of 0.5 M EDTA pH 8.0).

### 4.2 Taxonomic characterization of the bacterial strains

Strain CP5 was identified at the genus and species level by specific PCR techniques and by sequencing a segment of about 600 bp of ribosomal DNA (rDNA) 16S and at the strain level by PCR-RAPD techniques using the primers described in Table 5.

The primers Lm3 (SEQ.ID.NO:9) and Lm26 (SEQ.ID.NO:8) were used in the PCR specific for genus *Bifidobacterium.* The specific primers detailed in Table 5 were used for species PCR. The primers BIF (SEQ.ID.NO:16) and RFL-2 (SEQ.ID.NO:17) were used for the characterization at the strain level by means of PCR-RAPD. The primers 616V (SEQ.ID.NO:18) and 699R (SEQ.ID.NO:19) were used for amplifying and subsequently sequencing 16S rDNA.

Table 5. Sequences of the different primers used in the PCR and RAPD reactions.

**Table 5. Sequences of the different primers used in the PCR and RAPD reactions.**

| **Seq ID No** | **Primer** | **Sequence** | **Target** | **Amplified size** |
|---|---|---|---|---|
| 8 | Lm26 | | *Bifidobacterium* | 1350 bp |
| 9 | Lm3 | | | |
| 10 | BiBif-1 | | *B. bifidum* | 278 bp |
| 11 | BiBif-2 | | | |
| 12 | BiLON-1 | | *B. infantis* | 831 bp |
| 13 | BiLON-2 | | | |
| 14 | BiCAT-1 | 5'CGGATGCTCCGACTCCT'3 | *B. catenulatum* | 285 bp |
| 15 | BiCAT-2 | 5'CGAAGGCTTGCTCCCGAT'3 | | |
| 16 | BIF | 5'AGTCAGCCAC'3 | Universal | Several |
| 17 | RFL-2 | 5' CGGGCCCTGT'3 | Universal | Several |
| 18 | 616V | 5' AGA GTT TGA TYM TGG | Bacteria | 1500 bp |
| | | CTC AG 3' | | |
| 19 | 699R | 5' RGG GTT GCG CTC GTT 3' | | |

The PCR reaction mixture varied according to the type of analysis conducted. The different concentrations that were established for a reaction volume of 50 µl are shown in Table 6.

Table 6. Different reaction mixture concentrations for the PCR and RAPD reactions.

| | PCR genus | PCR species | RAPD | PCR 16S |
|---|---|---|---|---|
| gDNA | 200 ng | 200 ng | 200 ng | 200 ng |
| 10 X Buffer | 5 µl | 5 µl | 5 µl | 5 µl |
| Mg Cl₂ | 1.5 mM | 1.5 mM | 4 mM | 1.5 mM |
| Polymerase | 2U | 1.8 U | 2.5 U | 1 U |
| dNTPs | 300 µM | 200 µM | 200 µM | 200 µM |
| Primers | 1 µM | 0.5 µM | 1.5 µM | 1 µM |

The conditions in which the polymerization was performed also varied according to the type of analysis and are shown in Table 7.

**Table 7. Reaction conditions for PCR and RAPD reactions.**

| | | PCR genus | PCR species | RAPD | PCR 16S |
|---|---|---|---|---|---|
| Stages | Cycles | | | | |
| Start | 1 | | 94°C-5 min | 94°C-5 min | 94°C-5 min |
| | | | | | |
| | | 94°C-1 min | 94°C-20 s | 94°C-1 min | 94°C-20 s |
| Polymerization | 35 | 58°C-3 min | 58°C-20 s | 32°C-2 min | 56°C-30 s |
| | | 72°C-1 min | 72°C-30 s | 72°C-2 min | 72°C-1 min |
| | | | | | |
| Elongation | 1 | 72°C-4 min | 72°C-5 min | 72°C-2 min | 72°C-10 min |

Strain CP5 was identified by means of specific PCR as belonging to species *Bifidobacterium bifidum* (Figure 1). This identification was confirmed by means of comparing the 16S rDNA sequence of strain CP5 (SEQ,ID.NO: 7) with the total of the gene sequences included in public databases, obtaining 98% homology with the sequences deposited in public databases belonging to species *B. bifidum.*

### 5. Assays of resistance to gastrointestinal transit with simulated gastric and intestinal fluid systems

### 5.1. Resistance to gastrointestinal juices

Gastric and pancreatic juices were prepared at the moment of use by resuspending pepsin (Sigma) from porcine stomach mucosa (3 g/L) and pancreatin (Sigma) from porcine pancreas (1 g/L) in 0.5% sterile saline solution (w/v).

The bacterial strains were grown at 37°C for 24 hours in anaerobiosis, were washed twice in 0.5% saline solution and were taken to an inoculum of 10⁸-10⁹ cfu/mL.

The assay emulated the exposure times and conditions to which the bacteria are subjected during their transit through the gastric and intestinal tract in two stages. The first one simulated the gastric tract, for which purpose a 1% inoculum (v/v) of the bacteria was formed in 10 mL of saline solution with pepsin adjusted to pH 3.0 with HCl and it was incubated at 37°C in anaerobiosis for 120 minutes. The second one mimicked the transit through the intestinal tract, for which purpose the cells were neutralized by washing them with phosphate buffer (PBS) at pH 7.0 and they were resuspended in saline solution with pancreatin adjusted to pH 8.0 with NaOH, and incubated in anaerobiosis for 240 minutes. Samples were taken at 0, 90 and 120 minutes in the first stage and at 0, 60 and 240 minutes in the second stage, in each case making serial dilutions and seeding 100 µL of each dilution per plate of solid MRS-C medium and incubating them for two days at 37°C in anaerobiosis. The same strains were inoculated into saline solution as a viability control throughout the process and they were incubated in the same conditions taking aliquots at the start, at 120 minutes and at the end of the assay.

After incubation in the presence of gastric and intestinal juices, strain CP5 and the control *Lactobacillus* GG were recovered in plates with MRS-C medium, and the viable cells were counted after 48 hours of incubation. Both strains showed a similar loss of viability after exposure to the juices due to both the direct action of such juices and due to the extreme pH values (Table 8). The percentage of viability was calculated as the ratio of the number of viable cells obtained in each of the stages of the assay to the number of viable cells at time zero, which was the inoculum (10⁷-10⁸ cfu/mL)

**Table 8. In vitro effect of the gastrointestinal juices on the viability of the selected strains.**

| | | Pepsin pH 3, 120 min | | pancreatin pH 8, 240 min | |
|---|---|---|---|---|---|
| Strains | Initial inoculum cfu/mL | Count | % viable cells | Count | % viable cells |
| CP5 | 2.7 x 10⁸ | 2.5 x 10⁷ | 9.2% | 1.9 x 10⁷ | 7.0% |
| LGG | 3.4 x 10⁸ | 3.0 x 10⁷ | 8.8% | 1.0 x 10⁷ | 2.9% |

### 5.2. Resistance to bile salts and NaCl

The resistance of the two bacterial strains to different concentrations of bile salts, Ox-gall and NaCl, was analyzed. The assay was based on monitoring the growth of the bacteria in multiwell plates for 96 samples (Biorad). 100 µL of an inoculum at an OD₆₀₀ₙₘ of 0.1 unit and 175 µL of MRS-C medium supplemented with 0, 0.5, 1, 2 and 3% Oxgall (w/v), or MRS-C medium supplemented with 0, 2, 3, 6, 8 and 10% NaCL (w/v) were added to each well. The optical density of 24-hour cultures was analyzed at a wavelength of 655 nm in a microplate reader (Roche).

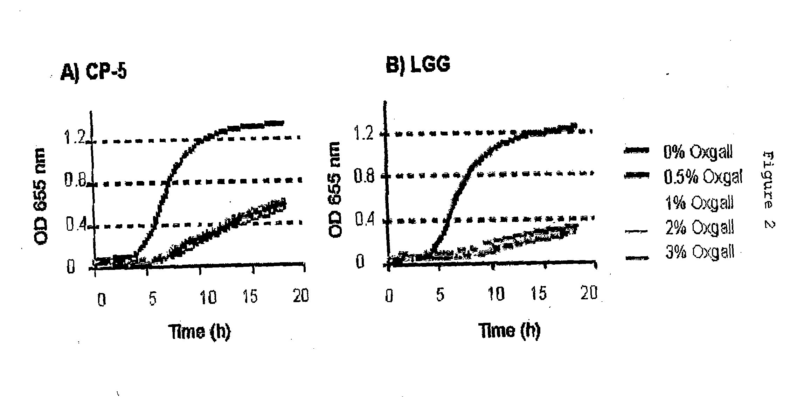

The tolerance of both strains at concentrations of 0.5, 1, 2 and 3% (w/v) of bile salts was analyzed by monitoring the growth in multiwell plates in these conditions by changing the optical density to 655 nm. The results show that the two strains were capable of growing in the presence of the different concentrations of bile salts, although CP5 showed a slightly higher resistance (Figure 2).

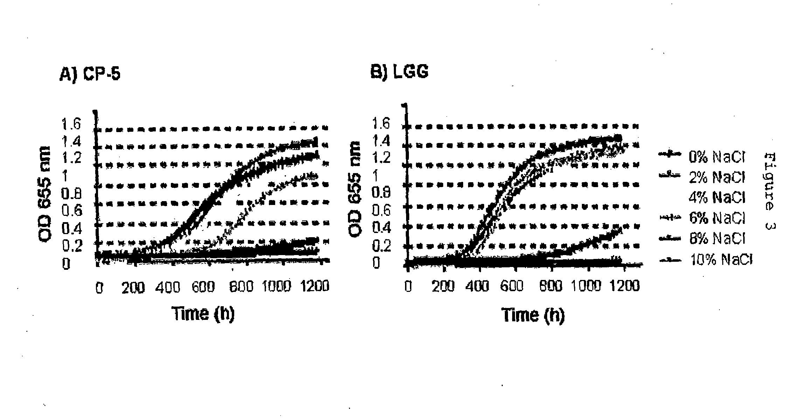

A study was performed to determine if these strains were resistant at high NaCl concentrations in the medium. Concentrations of 2, 3, 6, 8 and 10% (w/v) were analyzed for that purpose. The results indicated that both strains tolerated NaCl concentrations of up to 6%, a reduction of growth being observed from 8% NaCl, with a growth inhibition at the value of 10%. In this case, the strain *Lactobacillus* GG was slightly more resistant (Figure 3).

Bacterial growth studies at acidic pH values were performed. The assayed pHs were 1.5, 2.0 and 3.0 for an incubation period of 24 hours at 37°C in anaerobic conditions. The results indicated that growth inhibition occurred in the two assayed strains at these pHs. These data serve as indicators of the sensitivity of the strains to acidic pHs in *in vitro* assays for long periods of time (24 h). However, it must be considered that the *in vivo* conditions are different. On one hand, the dwell time of the probiotic in stomach areas where these pHs are reached does not typically exceed two hours and they quickly pass on to the small intestine where pH is neutralized, allowing bacterial growth. Furthermore, other compounds coming from the matrix of the food are ingested together with the probiotic which can act by buffering the inhibitory effect of the stomach pH. A study was therefore performed with greater pH range and at an incubation time of 2 hours, which is closer to the theoretical dwell time of the probiotic in the gastric system. 3 ml of suspension were taken from cultures obtained in a broth at 17 hours of growth and they were centrifuged at 4000 rpm for 5 minutes. The culture medium was removed and 10 ml of phosphate buffered saline (PBS) adjusted to each of the assayed pHs were added. After homogenization, they were incubated for 2 hours in a cabinet at 37°C. Counts i) of the initial culture; ii) of the mixture after 2 hours at 37°C and iii) of a control performed like in the other situations but in physiological conditions to assure that significant cell losses did not occur after centrifuging and decanting the culture medium were performed for each culture in MRS-C medium. No differences in the count results were observed between the initial culture and the control after 2 hours of incubation. The results obtained (Table 9) indicate a CP-5 survival very close to 100% at pH 4-7.

**Table 9. Counts obtained for strain CP-5 after 2 hours of incubation in buffer at different pHs.**

| | Count (cfu/ml) |
|---|---|
| pH2 | 1.00 x10² ± 0.00 |
| pH3 | 6.95 x10⁴ ± 4.17 x10⁴ |
| pH4 | 2.67 x10⁷± 9.40 x10⁶ |
| pH5 | 2.25 x10⁷ ± 7.07x10⁵ |
| pH6 | 2.08 x14⁷ ± 9.48x10⁶ |
| pH7 | 3.15 x10⁷± 2.83x10⁶ |
| Initial | 3.87 x10⁷ ± 1.41 x10⁶ |

### 6. Sensitivity to antibiotics

The sensitivity of the strains to antibiotics was determined by means of agar diffusion techniques using disks impregnated with various antibiotic concentrations. The bacteria grown in liquid MRS-C medium at 37°C for 24 hours were collected, washing them several times with saline solution and adjusting the inoculum to 10⁹ cfu/mL (OD₆₀₀nm = 5). 100 µL of the cell suspension were taken in these conditions and seeded in 10 mL of 0.7% semi-solid MRS-C agar (w/v) cooled to 50°C. This agar was poured onto a plate of MRS-C medium and was left to solidity at room temperature. Once dry, the antibiotic discs were placed on the surface. After 24 hours of incubation in anaerobiosis, the presence or absence of inhibiting areolas was observed.

A total of 17 antibiotics were tested to see the resistance or sensitivity of the different bacterial strains. The antibiotics used were nalidixic acid (30 µg), amoxicillin (25 µg), carbenicillin (100 µg), clarithromycin (15 µg), chloramphenicol (30 µg). erythromycin (15 µg), gentamicin (10 µg), kanamycin (30 µg), metronidazole (5 µg), oxacillin (1 µg), penicillin (6 µg), polymyxin B (300 IU), rifampicin (5 µg), sulfamide (200 µg), tetracycline (30 µg), trimethoprim (5 µg) and vancomycin (30 µg).

The sensitivity of the two strains to different types of antibiotics is shown in Table 10. This is one of the selection criteria of novel strains of probiotics, because by knowing their resistance profile, safety data is known for their use, which allows establishing the bases for a possible application as a complement in the antibiotic treatment of infections such as peptic ulcers caused by *H. pylori.*

The two strains showed resistance to eight of the seventeen assayed antibiotics, which included nalidixic acid, gentamicin, kanamycin, metronidazole, oxacillin, polymyxin, sulfamide and vancomycin. This sensitivity profile to antibiotics is described for other probiotic strains of the genus *Bifidobacterium* (*B. adolescentis, B. breve, B. infantis* and *B. longum*).

The resistance data for bifidobacteria to antibiotics such as gentamicin or metronidazole, which are typically used in treatments of *H. pylori* infections, indicate that it is possible to use the selected lactic acid bacteria in combined therapies which would include probiotic treatment with the administration of these antibiotics.

**Table 10. Resistance of the selected strains to different antibiotics.**

| | Diameter of the bacterial growth inhibition (mm) | | |
|---|---|---|---|
| Antibiotic | µg | CP5 | LGG |
| Amoxicillin | *25* | 28 | 30 |
| Carbenicillin | *100* | 36 | 32 |
| Oxacillin | *1* | 0 | 0 |
| Penicillin | *6* | 34 | 32 |
| Vancomycin | *30* | 0 | 0 |
| Chloramphenicol | *30* | 20 | 27 |
| Erythromycin | *15* | 27 | 29 |
| Gentamicin | *10* | 0 | 0 |
| Kanamycin | *30* | 0 | 0 |
| Tetracycline | *30* | 30 | 31 |
| Metronidazole | *5* | 0 | 0 |
| Nalidixic acid | *30* | 0 | 0 |
| Rifampicin | *5* | 34 | 32 |
| Trimethoprim | *5* | 0 | 0 |
| Polymyxin B | *300^{a}* | 0 | 0 |
| Sulfamide | *200* | 0 | 0 |
| Clarithromycin | *15* | 0 | 0 |

| | | | |
|---|---|---|---|
| ^{a}_{'} value expressed in IU | | | |

### 7. Adhesion assays for the adhesion to intestinal mucus

The assay was performed using the methodology used in point 3. Briefly, the strains CP5 and *Lactobacillus* GG grown in anaerobiosis for 24 hours at 37°C were washed twice with HEPES - Hanks buffer and the microorganism concentration was adjusted to OP_{600 nm} of 0.25 + 0.05. The cells were subsequently fluorescently labeled with CFDA and incubated at 37°C for 45 minutes, protecting the mixture from the light. 100 µl of labeled cells were added on the mucus immobilized in multiwell polystyrene plates (Maxisorp, Nunc) to each of the wells and they were incubated for 1 hour at 37°C. The bacteria that were not adhered were removed by means of two washings with of 200 µl of HH buffer. The bacteria that did adhere were released by means of scraping the mucus immobilized in each well and were subsequently lysed with 1% SDS (w/v) in 0.1 M NaOH (200 µl per well), incubating the plates at 60°C for 1 hour. The contents of the cells were transferred to a new multiwell polystyrene plate (Nunc) and the released fluorescence was measured at λₑₓ=485 nm; λₑₘ=518 nm in a Fluoreskan Ascent FL apparatus(Thermo). The adhesion was expressed as the percentage of fluorescence recovered after the adhesion in relation to the fluorescence of the bacterial suspension added to the immobilized mucus. The bacterial adhesion was determined in three independent experiments and each assay was performed in quadruplicate.

The two strains were subjected to an adhesion assay for the adhesion to type III porcine mucus samples. Strain CP5 showed 12% adhesion versus 10% of the strain *Lactobacillus* GG. 8. Production and purification of the metabolite of interest.

A fermentation of 10 liters with strain CP-5 in MRS medium supplemented with cysteine (0.05% w/v) was carried out for the purpose of obtaining a supernatant rich in the metabolite responsible for inhibition of *H. pylori.* The incubation was carried out at 37°C for 17 hours without stirring and with an anaerobiosis generator, a cell density of the culture of 1.25-10⁸ cfu/ml being obtained. The amount of inoculum was 1% of the total fermentation volume. Finally, the supernatant was obtained by centrifugation at 10000 rpm for 15 minutes and it was frozen at -80°C until the purification process. 8.1. Purification of the metabolite of interest by cation-exchange chromatography followed by another stage of reversed-phase chromatography

200 ml of supernatant were subjected to a stage of cation-exchange chromatography with the HiPrep 16/10 SP FF column (GE Healthcare). In this stage, all the cationic proteins and peptides were adsorbed to the resin and were finally eluted with a buffer with 1M NaCl without gradient. All the cationic proteins eluted simultaneously in these conditions and they were collected in a few fractions (fractions 3, 4 and 5) and in a minimum volume to concentrate the metabolite of interest. The volume of each fraction was 10 ml. The percentage of inhibition of *H. pylori* in these fractions was 50.32%, 81.29% and 1.9%, respectively. Cationic exchange fraction 4, which presented the highest inhibition value, was subjected to an ultrafiltration process with 5000 Da filters (Extreme Amicon, Millipore). The filtered volume of this stage contained the proteins under this molecular weight. This filtered volume was subjected to a stage of reversed-phase chromatography to purify and subsequently identify by MALDI-TOF mass spectrometry the metabolite responsible for inhibiting *H. pylori.* For this stage, a RESOURCE RPC 3 ml column (GE Healthcare) was used and a gradient as worked with containing the following eluents: A) Milli-Q water + 0.1% trifluoroacetic acid (TFA) and B) acetonitrile + 0.1% trifluoroacetic acid (TFA). In this chromatographic stage, 2 ml fractions were obtained. An aliquot of these fractions was dried to remove the solvent and was finally resuspended in distilled water right before testing the inhibition of *H. pylori* by means of these fractions. Fraction 11 obtained by reversed-phase chromatography presented an inhibition value of *H.Pylori* of 94.77%, the following peptides, all of which are from bovine β-casein, being identified in this fraction:

| | |
|---|---|
| SEQ. ID. NO: 1 VYPFPGPIHN | 1139.57 Da |
| SEQ. ID. NO: 2 PPFLQPEVMGVSKVKE | 1783.95 Da |
| SEQ. ID. NO: 3 DKIHPFAQTQSLVYPF | 1889.97 Da |
| SEQ. ID. NO: 4 PYPQRDMPIOAFLLYQ | 1978.99 Da |
| SEQ. ID. NO: 5 RDMPIQAFLLYQEPVLG | 1978.99 Da |
| SEQ. ID. NO: 6 PQNIPPLTQTPVVVPPFLQPE | 2310.26 Da |

### 9. Metabolic tests

### 9.1 Determination of lactic acid (D/L)

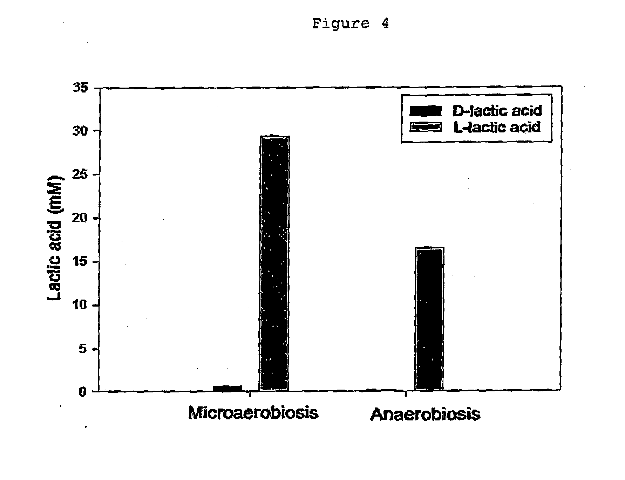

To quantify the lactic acid (D/L) in the culture supernatant of strain CP-5, the commercial D-Lactic acid/L-Lactic acid Kit (Roche, Cat. No. 11 112 821 035) was used. This determination consists of the spectrophotometric analysis of the product generated by the specific action of two enzymes (D-lactate dehydrogenase and L-lactate dehydrogenase) on both of the lactic acid isomers. The lactic acid (D/L) was quantified in culture supernatants of strain CP-5 grown at 37°C in microaerobic and anaerobic conditions. The results of the analysis are shown in Figure 4.

### 9.2 Deconjugation of bile salts.

The hydrolysis of bile salts releasing free amino acids and cholesterol were carried out by the action of the bile salt hydrolase (BSH, EC 3.5.1.24) enzyme. The BSH enzyme activity assay was carried out as described in the article by Kumar *et al*. (2006)⁴⁴. This article proposes determining the BSH enzyme activity on two different substrates, two bile salts the amino acids of which are glycine and taurine. The reaction of these amino acids with a ninhydrin solution gives a colored compound which is spectrophotometrically determined at 570 nm. A standard curve is previously made for both glycine and for taurine.

The BSH activity was determined in culture supernatants of strain CP-5 grown in microaerobic and anaerobic conditions. Before determining the BSH activity, the standard curves for glycine and taurine were made. Table 11 shows the BSH activity values for strain CP-5.

**Table 11. BSH activity for strain CP-5 versus different substrates.**

| | BSH-Taurine (mlU/ml) | BSH-Glycine (mlU/ml) |
|---|---|---|
| Microaerobiosis | 5.25 ± 0.12 | 4.45 ± 0.40 |
| Anaerobiosis | N.D. | N.D. |

| | | |
|---|---|---|
| N.D.: BSH activity was not detected in the assay conditions. | | |

### 9.3 Determination of biogenic amines

To quantify biogenic amines in the culture supernatant of strain CP-5, a chromatographic method (HPLC) described by Eerola *et al.* (1993)⁴⁵ was used in which the biogenic amines present in the supernatant were previously derivatized with a dansyl chloride solution. The biogenic amines determined were tyramine, histamine, cadaverine and putrescine. A standard curve was previously made with the four biogenic amines analyzed.

To quantify biogenic amines in the culture supernatant of strain CP-5, standard curves for the amines tyramine, histamine, cadaverine and putrescine were made. Table 12 shows the amount of biogenic amines produced in the culture supernatants of strain CP-5 cultured in microaerobic and anaerobic conditions.

**Table 12. Biogenic amines produced by strain CP-5.**

| | Putrescine (µm) | Cadaverine (µM) | Histamine (µM) | Tyramine (µM) |
|---|---|---|---|---|
| Microaerobiosis | N.D. | 0.0656±0.0000 | N.D. | 0.7815±0.0816 |
| Anaerobiosis | N.D. | 0.0851±0.0382 | N.D. | 0.8179±0.0904 |

| | | | | |
|---|---|---|---|---|
| N.D.: Not detected in the assayed supernatant. | | | | |

The results indicate that these parameters are within the normal levels and allowed for other probiotic bacteria.

### 10. Uses of the novel strain of CP5 of Bifidobacterium bifidum

### 10.1 Obtaining culture supernatant from CP5 bifidobacteria.

Starting from CP5 bifidobacteria glycerinated extract, a product referred to as CP5 bifidobacteria cell supernatant is obtained in about 5 days.

Day 1: Seeding glycerinated extract with a loop (10 µl) of bifidobacteria in 10 mL of MRS-C broth (Annex 1) and MRS-C plate. Growth for 17 hours (broth) and 30 hours (plate) in a laboratory cabinet at 37°C with an anaerobiosis generator.

Day 2: Checking the purity and the condition of the culture from macroscopic viewing of colonies and microscopic viewing of the cells. Passage 1 of the culture (pre-inoculum). Seeding 100 µl of the culture (1% v/v) in 10 mL of MRS-C broth. Growing for 17 hours in a laboratory cabinet at 37°C.

Day 3: Passage 2 of the culture (inoculum). Seeding 2 mL of the culture of passage 1 in a 200 mL flask with MRS-C. Growing for 17 hours in a laboratory cabinet at 37°C. Preparing a 20 L carboy in non-commercial MRS-C medium and sterilization in autoclave.

Day 4: Taking a sample of the inoculum. MRS-C plate counts from seeding at least three serial decimal dilutions (-6, -7 and -8) in 0.09% NaCl saline solution pH 7.0. Growing for 30 hours in a laboratory cabinet at 37°C with an anaerobiosis generator. Measuring OD and dry weight (Annex 1). Inoculating the carboy. 200 mL of inoculum (1%) are inoculated in maximum (flash) sterility conditions. Growing for 17 hours in a controlled temperature chamber at 37°C.

Day 5: Taking a sample of the production in sterility. Checking the purity and the condition of the culture from microscopic viewing of the cells. Measuring OD and dry weight (Annex 1). MRS-C plate counts of the product from seeding at least three serial decimal dilutions (-6, -7 and -8) in 0.09% NaCl saline solution pH 7.0. Growing for 30 hours in a laboratory cabinet at 37°C with an anaerobiosis generator. Recovering the cells by means of centrifugation in a Westfalia centrifuge (input rate of 48 liters/hour). The collected cells are resuspended in 400 mL of 0.09% NaCl saline solution pH 7.0. The product is centrifuged at 4000 rpm for about 30 minutes (depending on the sedimentation condition, the centrifugation will be increased by 15 minutes). The saline solution is decanted. This step is performed in duplicate.

The washed cells are resuspended in a volume of 1500 ml of cryoprotectant solution.

### Annex 1:

### A. Composition of the MRS-C medium (pH = 6.5).

- 10.00 g/L of casein peptone (Fluka)
- 10.00 g/L of meat extract (Pronadisa)
- 5.00 g/L of yeast extract(Pronadisa)
- 20.00 g/L of glucose (Merck)
- 1.00 g/L of Tween 80 (Fluka)
- 2.00 g/L of K₂HPO₄ (Panreac)
- 5.00 g/L of sodium acetate (Scharlau)
- 2.00 g/L of (NH₄)₂ citrate (Fluka)
- 0.20 g/L of MgSO₄ x 7 H₂O (Panreac)
- 0.05 g/L of MnSO₄ x H₂O (Merck)
- 0.05 g/L* of cysteine (Panreac)
- 15 g/L of industrial agar (Pronadisa)
Autoclave: 121°C for 20 minutes in production autoclave.

The cysteine is prepared separated in a 100x solution and is added to the already sterilized medium.

The agar is only added in the case of solid medium.

### 10.2. Obtaining a lyophilisate of the novel strain CP5 of Bifidobacterium bifidum

The objective is to obtain a lyophilized product of bifidobacteria with a concentration equal to or greater than 10¹⁰ cfu/g.

Day 6: Starting from the washed cells obtained from production (see point 10), the cells are resuspended in the cryoprotectant solution, made up of 1500 ml UHT fluid skim milk and 5% food-grade sucrose. The product is distributed in lyophilization trays and frozen at -80°C.

Day 7: Lyophilization. Dehydrating by sublimation is performed in a Christ Beta 2-16 model lyophilizer in two phases:
A) Primary drying: Temperature of the condenser: - 80°C. Temperature of the tray: 0°C. Pressure in the drying chamber: 0.250 mbar. Safety pressure: 0.420 mbar. Drying time: 66 hours.
B) Final Drying: Temperature of the condenser: - 80°C. Temperature of the tray: equal to room temperature, between 20°C and 25°C. Pressure in the drying chamber: 0.050 mbar. Drying time: 2 hours.

Day 8: Collecting the lyophilization product. Weighing and measuring Aw and moisture. MRS-C plate counts of the product from seeding at least three serial decimal dilutions (-6, -7 and -8) in 0.09% NaCl saline solution pH 7.0. Growing for 30 hours in a laboratory cabinet at 37°C with an anaerobiosis generator. Vacuum-packing the product in the least time possible. Storing at 4°C until delivery.

### 10.3. Use of CP5 as probiotic

### - Use of CP5 in foods

A probiotic food is any food which contains live microorganisms present in a food which remain active in the intestine and have important physiological effects. When ingested in sufficient amounts, they have a very beneficial effect, such as contributing to the equilibrium of the host's intestinal bacterial flora and enhancing the host's immune system. They are not pathogens. Fresh yogurts, other fermented milk, etc., contain microorganisms of this type and are therefore probiotic foods.

One of the benefits of probiotic foods is the improvement of the balance of intestinal flora. Foods referred to as probiotics are simple or mixed cultures of probiotic bacteria which, when consumed both by humans and by animals, survive the passage through the gastrointestinal tract and are (not necessarily) implanted in the colon or small intestine, being capable of adhering to the intestinal mucosa, favorably affecting the host in terms of improved health. The reiterated consumption of probiotic yogurt in relatively abundant amounts has a therapeutic effect *against H. pylori* (Guilliland, 1998)⁴⁶.

Today, probiotics are used both In milk and in cheeses, pasteurized juices, ice-cream, yogurts, cereals and nutrition bars. Since probiotics are live organisms, their viability is a key point for their success.

The cells of strain CP5 maintain viability of 88% for at least a period of 15 days at 5°C when added to a dairy product.

Strain CP5 can be used as a probiotic by adding it to foods in general and particularly to dairy products and yogurts, both directly as fermented products, and in this case also directly as a lyophilisate.

An example of the use of CP5 in the form of a lyophilisate obtained in point 11 would be to obtain a dietetic composition, for example from pasteurized milk, to which the lyophilisate containing preferably 10⁵-10¹¹ cfu/g of CP5 cells is added, which are mixed with the pasteurized milk, and the mixture is homogenized at about 5°C and the milk thus homogenized is bottled and packaged as is usual for consumption thereof. In this case the product has a duration of at least 15 days at 5°C.

### - Use of CP5 in pharmaceutical compositions

The lyophilisate of CP5 obtained in point 11 is used, and it is ground to a maximum particle size of 1 mm in diameter and is mixed in a physiologically effective amount, which will generally contain 10⁹-10¹¹ cfu/g of cells of CP5, with the substances necessary for forming pills, capsules, etc. A physiologically effective amount is the amount of an active agent necessary in order to achieve the desired effect, whether it is to prevent or cure, the latter being the case of *H, pylori* infection.

The pharmaceutical compositions of the invention can include one or more pharmaceutically acceptable excipients, adjuvants, diluents or carriers which are generally known by the person skilled in the art.

The pharmaceutical compositions of the invention can be prepared in solid form, such as for example, pills, pellets tablets, powders, etc.; in liquid form, such as for example by mixing the active ingredient with water, oils or mixtures of both, in suspensions or dispersions mixed with dispersing and/or suspension agents, such as for example gums, etc.

The lyophilisate of CP5 can also be administered in pharmaceutical compositions together with antibiotics for treating gastric ulcers and preventing stomach cancer.

In both cases, in pharmaceutical and dietetic or nutritional compositions, the use of said compositions will be to prevent or fight infections caused by *H. pylori* in gastrointestinal diseases, among others, stomach ulcers and stomach cancer.

### LITERATURE

1. Bizzozero, G. (1892). "Ueber die schlauchförmigen Drüsen des Magendarmkanals und die beziehungen ihres Epitheles zu dem Oberflächenepithel der Schleimhaut". Archiv für mikroskopishe Anatomie. 42: 82-152.
2. Marshall BJ (1983). "Unidentified curved bacillus on gastric epithelium in active chronic gastritis". Lancet 1 (8336): 1273-75.
3. Marshall BJ & Warren JR (1984). "Unidentified curved bacilli in the stomach patients with gastritis and peptic ulceration". Lancet 1 (8390): 1311-1315
4. Gasbarrini, G. et al. (2000) "No evidence of Helicobacter pylori sequences in pancreatic juices of patients affected by chronic pancreatitis" Int J Pancreatol. 28 (3): 181-5.
5. Gisbert, S. et al. (2004) "Meta-analysis: Helicobacter pylori eradication therapy vs. antisecretory non-eradication therapy for the prevention of recurrent bleeding from peptic ulcer" Aliment Pharmacol Ther. 19: 617-29.
6. Taylor DN & Blazer MJ (1991) "The epidemiology of Helicobacter pylori infection" Epidemiol Rev. 13: 42-59.
7. Peterson WL (1991) "Pathogenesis and therapy of peptic ulcer disease" J Clin Gastroenterol. 12 suppl 2: S1-6.
8. Solnick JV, Tompkins LS (1992) "Helicobacter pylori and gastroduodenal disease: pathogenesis and host-parasite interaction" Infect Agents Dis. 1 (6): 294-309.
9. The EUROGAST Study Group "An international association between Helicobacter pylori and gastric cancer". (1993) Lancet 341: 1359-62.
10. Chong J et al. (1994) "Occupational exposure to Helicobacter pylori for the endoscopy professional: a sera epidemiological study" Am J Gastroenterol. 89 (11): 1987-92.
11. Vilella, A. et al. (2003) "Linfoma gástrico tipo MALT" Med Clin (Barc) 120 (9): 349-52.
12. Fox, J.G. & Wang, T.C. (2001) "Helicobacter pylori-not a good bug after all!" New Engl. J. Med. 345:829-832.
13. Lind T., et al. (1996) "Eradication of Helicobacter pylori using one-week triple therapies combining omeprazole with two antimicrobials: the MACH I Study" Helicobacter. 1 (3): 138-44.
14. The European Helicobacter pylori Study Group (1997) "The year in Helicobacter pylori 1997: Current Opinion in Gastroenterology" (Book) vol. 13, supplement 1.
15. Deltenre M. et al. (1998) "Eradication of Helicobacter pylori: why does it fail?" Ital J Gastroenterol Hepatol. 30 Supplement 3: 326-8.
16. Graham DY, Lew GM, Evans DG, Evans DJ, Klein PD (1991). "Effect of triple therapy (antibiotics plus bismuth) on duodenal ulcer healing. A randomized controlled trial". Ann. Intem. Med. 115 (4): 266-9.
17. Bhatia SJ et al. (1989) "Lactobacillus acidophilus Inhibits Growth of Campylobacter pylori In Vitro" Journal of Clinical Microbiology p.2328-2330.
18. Mrda, Z et al. (1998) "Therapy of Helicobacter pylori infection using Lactobacillus acidophilus" Med Pregi. 51 (7-8): 343-5.
19. Gasbarrini et al. (2004) "A lyophilized and inactivated culture of Lactobacillus acidophilus increases Helicobacter pylori eradication rates"
20. Lorca, GL et al. (2001) "Lactobacillus acidophilus Autolysins Inhibit Helicobacter pylori In Vitro" Current microbiology vol.42, 39-44.
21. Coconnier M-H et al. (1998) "Antagonistic Activity against Helicobacter infection In Vitro and In Vivo by the Human Lactobacillus acidophilus Strain LB" Applied and Environmental Microbiology, p. 4573-80.
22. Michetti P. et al. (1999) "Effect of whey-based culture supernatant of Lactobacillus acidophilus (johnsonii) La1 on Helicobacter pylori infection in humans" Digestion 60 (3): 203-9.
23. Sgouras D. et al. (2004) "In vitro and in vivo inhibition of Helicobacter pylori by Lactobacillus casei strain Shirota" Appl Environ Microbiol. 70 (1): 518-26.
24. Johnson-Henry KC et al. (2004) "Probiotics reduce bacterial colonization and gastric inflammation in H. pylori-infected mice" Dig Dis Sci. 49 (7-8): 1095-102.
25. Hamilton-Miller JM (2003) "The role of probiotics in the pretreatment and prevention of Helicobacter pylori infection" Int J Antimicrob Agents 22 (4): 360-6.
26. Pinchuk IV. et al., (2001) "In vitro anti-Helicobactyer pylori activity of the probiotic strain Bacillus subtilis is due to secretion of antibiotics" Antimicrob Agents Chemother. 45 (11): 3156-61.
27. Nam et al. (2002) "Effect of Weissella confusa strain PL9001 on the adherence and growth of Helicobacter pylori" Appl. Environ. Microbiol. 68:4642-4645.
28. Ishihara K et al. (1997) "Oral bacterium inhibit Helicobacter pylori growth" FEMS Microbiol Lett. 15:152(2):355-61.
29. Aiba Y. et al. (1998) "Lactic acid-mediated suppression of Helicobacter pylori by the oral administration of Lactobacillus salivarius as a probiotic in a gnotobiotic murine model" Am J Gastroenterol. 93 (11): 2097-101.
30. Kabir AM et al. (1997) "Prevention of Helicobacter pylori infection by lactobacilli in a gnotobiotic murine model" Gut. Jul. 41(1):49-55.
31. Ushiyama A. et al. (2003) "Lactobacillus gasseri OLL2716 as a probiotic in clarithromycin-resistant Helicobacter pylori infection" J Gastroenterol Hepatol. 18 (8): 986-91.
32. Mukai M. et al. (2002) "Helicobacter pylori associated with idiopathic thrombocytopenic purpura" Am J Med 113(2): 169-71.
33. Armuzzi A. et al. (2001) "Effect of Lactobacillus GG supplementation on antibiotic-associated gastrointestinal side effects during Helicobacter pylori eradication therapy: a pilot study" Digestion 63 (1): 1-7.
34. Armuzzi A. et al. (2001) "The effect of oral administration of Lactobacillus GG on antibiotic-associated gastrointestinal side-effects during Helicobacter pylori eradication therapy" Aliment Pharmacol Ther. 15 (2): 163-9.
35. Felley CP. et al. (2001) "Favourable effect of an acidified milk (LC-1) on Helicobacter pylori gastritis in man" Eur J Gastroenterol Hepatol. 13 (1): 25-9.
36. Cremonini F. et al. (2001) "Effect of different probiotic preparations on anti-Helicobacter pylori therapy-related side effects: a parallel group, triple bind, placebo-controlled study" Am J Gastroenerol 97 (11): 2744-9.
37. Canducci F. et al. (2000) "A lyophilized and inactivated culture of Lactobacillus acidophilus increases Helicobacter pylori eradication rates" Aliment Pharmacol Ther. 14 (12): 1625-9.
38. Of Francesco V et al. (2000) "Lactobacillus acidophilus administration added to omeprazole/amoxicillin-based double therapy in Helicobacter pylori eradication". Dig Liver Dis. 32(8): 746-7.
39. Sheu BS. et al. (2002) "Impact of supplement with Lactobacillus-and Bifidobacterium-containing yogurt on triple therapy for Helicobacter pylori eradication" Aliment Pharmaco Ther. 16 (9): 1669-75.
40. Mrda Z et al. (1993) "Helicobacter pylori in patients with diseases of the upper part of the gastrointestinal tract" Med. Pregl. 46(3-4):117-9.
41. Scholz-Ahrens, KE & Schrezenmeir J. et al. (2000) "Effects of bioactive substances in milk on mineral and trace element metabolism with special reference to casein phosphopeptides" Br J Nutr. 84 Suppl 1: 147-53.
42. Nakaji S et al. (2001) "Relationship between mineral and trace element concentrations in drinking water and gastric cancer mortality in Japan.". Nutr Cancer. 40(2):99-102.
43. Wendakoon CN. et al. (2002) "Lack of therapeutic effect of a specially designed yogurt for the eradication of Helicobacter pylori infection" Digestion. 65 (1): 16-20.
44. Kumar R.S. et al. (2006) "Structural and functional analysis of a conjugated bile salt hydrolase from Bifidobacterium longum reveals an evolutionary relationship with penicillin V acylase." J. Biol. Chem. 281: 32516-32525.
45. Eerola, S. et al. (1993) "Liquid chromatographic determination of biogenic amines in dry sausages". Int. J. AOAC 76: 575-577.
46. Guilliland SE. (1998) Fermented milks and probiotics. In: Applied Dairy Microbiology (Marth EH and Steele JL, eds.) Maarcel Dekker, Inc., New York, pp. 195-212.

## Claims

1. Strain of *Bifidobacferium bifidum* deposited in the Spanish type Culture Collection of Microorganisms (CECT) with accession number CECT 7366, with activity for inhibiting *Helicobacter pylori,* and having the capacity to produce six peptides **characterized in that** their amino acid sequence is SED.ID.NO: 1 to 6.

2. Bacterial strain of *Bifidobacterium bifidum* according to claim 1, **characterized in that** the bacteria are present as viable cells.

3. Bacterial strain of *Bifidobacterium bifidum* according to claim 1, **characterized in that** the bacteria are present as non-viable cells.

4. Bacterial culture, **characterized in that** it has been obtained from bacterial strain CECT 7366 according to any of claims 1-3.

5. Bacterial culture, **characterized in that** it comprises viable cells of bacterial strain CECT 7366, according to claims 1 and 2.

6. Bacterial culture, **characterized in that** it comprises non-viable cells of bacterial strain CECT 7366, according to claims 1 and 3.

7. Culture supernatant of bacterial strain CECT 7366 according to claims 1 to 3, obtained by fermentation, having anti - *H. pylori* activity.

8. Use of the strain of *Bifidobacterium bifidum* according to claims 1 and 2 for obtaining the peptides SEQ.ID.NO: 1 to 6.

9. Lyophilisate, **characterized in that** it has been obtained from bacterial strain CECT 7366 according to any of claims 1 to 3.

10. Lyophilisate according to claim 9, **characterized in that** it contains from 10⁵ - 10¹¹ cfu/g of bacterial strain cells CECT 7366, according to claims 1 and 3.

11. Use of the strain of *Bifidobacterium* CECT 7366 *bifidum* according to claims 1-3 for preparing a nutritional and/or pharmaceutical composition, **characterized in that** said bacterium and the peptides SEQ.ID.NO: 1-6 are present in an amount which is sufficient to have a probiotic effect on said nutritional and/or pharmaceutical composition that an individual needs, preferably against *Helicobacter pylori.*

12. Nutritional composition, comprising an effective amount of the strain *Bifidobacterium bifidum* CECT 7366 according to claims 1 - 3 or the strain *Bifidobacterium bifidum* CECT 7366 according to claims 1- 3 and the peptides with amino acid SEQ.ID.NO: 1 to 6, together with suitable amounts of other foods.

13. Nutritional composition according to claim 12, wherein the other foods are selected from the group comprising ice-cream, cereals, juices, nutrition bars, dairy products such as whole milk, skim milk, fermented milk, powdered milk, cheese, yogurts or other dairy products.

14. Nutritional composition according to claim 13, **characterized in that** it is a yogurt.

15. Yogurt composition according to claims 14, **characterized in that** the cells of the strain *Bifidobacterium bifidum* CECT 7366 according to claims 1- 3 have a viability of at least 80% for a period of 15 days when maintained at 5°C.

16. Nutritional composition according to claims 12-15, **characterized in that** the strain *Bifidobacterium bifidum* CECT 7366 according to claims 1- 3 is present in an amount between 10⁵- 10¹¹ cfu/g of the composition.

17. Strain of *Bifidobacterium bifidum* CECT 7366 according to claims 1-3 for use in preventing or curing gastric ulcers or stomach cancer caused by *Helicobacter pylori* infection.

18. Pharmaceutical composition comprising, a lyophilisate according to claims 9 to 10 in a physiologically effective amount, together with the necessary pharmaceutically acceptable carriers and/or adjuvants for preventing or curing gastric ulcers or stomach cancer caused by *Helicobacter pylori* infection.

19. Pharmaceutical composition according to claims 18, **characterized in that** said composition is in solid form, such as, for example, pills, tablets, etc; in liquid form, in suspension or dispersion.

20. Pharmaceutical composition according to claims 18 - 19, **characterized in that** it additionally comprises antibiotics for treating gastric ulcers.

21. Pharmaceutical composition according to claims 18 - 20, **characterized in that** the strain is present in an amount between 10⁷- 10¹¹ cfu/g.

## Patentansprüche

1. *Bifidobacterium bifidum-Stamm,* der in der spanischen Kultursammlung von Mikroorganismen (CECT) mit der Zugangsnummer CECT 7366 hinterlegt ist, mit Aktivität zur Hemmung von *Helicobacter pylori* und mit der Fähigkeit, sechs Peptide zu erzeugen, die **dadurch gekennzeichnet sind, dass** ihre Aminosäuresequenz SEQ. ID. Nr.: 1 bis 6 ist.

2. *Bifidobacterium bifidum*-Bakterienstamm nach Anspruch 1, **dadurch gekennzeichnet, dass** die Bakterien als lebensfähige Zellen vorhanden sind.

3. *Bifidobacterium bifidum*-Bakterienstamm nach Anspruch 1, **dadurch gekennzeichnet, dass** die Bakterien als nicht lebensfähige Zellen vorhanden sind.

4. Bakterienkultur, **dadurch gekennzeichnet, dass** sie aus dem Bakterienstamm CECT 7366 nach einem der Ansprüche 1-3 erhalten wurde.

5. Bakterienkultur, **dadurch gekennzeichnet, dass** sie lebensfähige Zellen aus dem Bakterienstamm CECT 7366 nach den Ansprüchen 1 und 2 umfasst.

6. Bakterienkultur, **dadurch gekennzeichnet, dass** sie nicht lebensfähige Zellen aus dem Bakterienstamm CECT 7366 nach den Ansprüchen 1 und 3 umfasst.

7. Kulturüberstand des Bakterienstamms CECT 7366 nach den Ansprüchen 1 bis 3, der durch Fermentation erhalten wird und Anti-*H*. *pylori-*Aktivität aufweist.

8. Verwendung des *Bifidobacterium bifidum-Stamms* nach den Ansprüchen 1 und 2 für den Erhalt der Peptide mit SEQ. ID. Nr.: 1 bis 6.

9. Lyophilisat, **dadurch gekennzeichnet, dass** es aus dem Bakterienstamm CECT 7366 nach einem der Ansprüche 1 bis 3 erhalten wurde.

10. Lyophilisat nach Anspruch 9, **dadurch gekennzeichnet, dass** es 10⁵- 10¹¹ KBE/g Bakterienstammzellen CECT 7366 nach den Ansprüchen 1 und 3 enthält.

11. Verwendung des *Bifidobacterium bifidum-Stamms* CECT 7366 nach Ansprüchen 1-3 für die Herstellung einer Nährstoff- und/oder pharmazeutischen Zusammensetzung, **dadurch gekennzeichnet, dass** das genannte Bakterium und die Peptide mit SEQ. ID. NR.: 1-6 in einer Menge vorhanden sind, die ausreichend ist, um eine probiotische Wirkung, vorzugsweise gegen *Helicobacter pylori,* bei der genannten Nährstoff- und/oder pharmazeutischen Zusammensetzung, welche ein Individuum benötigt, herbeizuführen.

12. Nährstoffzusammensetzung, umfassend eine wirksame Menge des *Bifidobacterium bifidum*-Stamms CECT 7366 nach Ansprüchen 1-3 oder des *Bifidobacterium bifidum*-Stamms CECT 7366 nach Ansprüchen 1-3 und der Peptide mit Aminosäure SEQ. ID. NR.: 1 bis 6, zusammen mit geeigneten Mengen anderer Lebensmittel.

13. Nährstoffzusammensetzung nach Anspruch 12, wobei die anderen Lebensmittel aus der Gruppe umfassend Eiscreme, Müsli, Säfte, Nährstoffriegel, Milchprodukte wie Vollmilch, entrahmte Milch, fermentierte Milch, Milchpulver, Käse, Joghurts oder andere Milchprodukte ausgewählt sind.

14. Nährstoffzusammensetzung nach Anspruch 13, **dadurch gekennzeichnet, dass** diese ein Joghurt ist.

15. Joghurtzusammensetzung nach Anspruch 14, **dadurch gekennzeichnet, dass** die Zellen des *Bifidobacterium bifidum*-Stamms CECT 7366 nach Ansprüchen 1-3 eine Lebensfähigkeit von mindestens 80 % für einen Zeitraum von 15 Tagen haben, wenn sie bei 5 °C gehalten werden.

16. Nährstoffzusammensetzung nach Ansprüchen 12-15, **dadurch gekennzeichnet, dass** der *Bifidobacterium bifidum*-Stamm CECT 7366 nach Ansprüchen 1-3 in einer Menge zwischen 10⁵-10¹¹ KBE/g der Zusammensetzung vorhanden ist.

17. *Bifidobacterium bifidum-*Stamm CECT 7366 nach Ansprüchen 1-3 zur Verwendung bei der Prävention oder Heilung von durch *Helicobacter pylori-*Infektion verursachten Magengeschwüren oder Magenkrebs.

18. Pharmazeutische Zusammensetzung, umfassend ein Lyophilisat nach Ansprüchen 9 bis 10 in einer physiologisch wirksamen Menge zusammen mit den notwendigen pharmazeutisch akzeptablen Trägerstoffen und/oder Adjuvanzien zur Prävention oder Heilung von durch *Helicobacterpylori-*Infektion verursachten Magengeschwüren oder Magenkrebs.

19. Pharmazeutische Zusammensetzung nach Anspruch 18, **dadurch gekennzeichnet, dass** die genannte Zusammensetzung in fester Form, wie zum Beispiel Pillen, Tabletten usw.; in flüssiger Form, in Suspension oder Dispersion vorliegt.

20. Pharmazeutische Zusammensetzung nach Ansprüchen 18-19, **dadurch gekennzeichnet, dass** sie außerdem Antibiotika zur Behandlung von Magengeschwüren umfasst.

21. Pharmazeutische Zusammensetzung nach Ansprüchen 18-20, **dadurch gekennzeichnet, dass** der Stamm in einer Menge zwischen 10⁷-10¹¹ KBE/g vorhanden ist.

## Revendications

1. Souche de *Bifidobacterium bifidum* déposée dans la Collection espagnole de cultures type (CECT) de microorganismes avec un numéro d'accès CECT 7366, avec une activité pour inhiber *Helicobacter pylori,* et ayant la capacité de produire six peptides **caractérisés en ce que** leur séquence d'acide aminé est SED.ID.NO : 1 à 6.

2. Souche bactérienne de *Bifidobacterium bifidum* selon la revendication 1, **caractérisée en ce que** les bactéries sont présentes comme des cellules viables.

3. Souche bactérienne de *Bifidobacterium bifidum* selon la revendication 1, **caractérisée en ce que** les bactéries sont présentes comme des cellules non-viables.

4. Culture bactérienne, **caractérisée en ce qu'**elle a été obtenue à partir d'une souche bactérienne CECT 7366 selon l'une quelconque des revendications 1-3.

5. Culture bactérienne, **caractérisée en ce qu'**elle comprend des cellules viables d'une souche bactérienne CECT 7366, selon les revendications 1 et 2.

6. Culture bactérienne, **caractérisée en ce qu'**elle comprend des cellules non-viables d'une souche bactérienne CECT 7366, selon les revendications 1 et 3.

7. Surnageant de culture d'une souche bactérienne CETC 7366 selon les revendications 1 à 3, obtenu par fermentation, ayant une activité anti-*H*. *pylori.*

8. Utilisation de la souche de *Bifidobacterium bifidum* selon les revendications 1 et 2 pour obtenir les peptides SEQ.ID.NO : 1 à 6.

9. Lyophilisat, **caractérisé en ce qu'**il a été obtenu à partir d'une souche bactérienne CECT 7366 selon l'une quelconque des revendications 1 à 3.

10. Lyophilisat selon la revendication 9, **caractérisé en ce qu'**il contient entre 10⁵-10" ufc/g de cellules d'une souche bactérienne CECT 7366, selon les revendications 1 et 3.

11. Utilisation de la souche de *Bifidobacterium* CECT 7366 *bifidum* selon les revendications 1-3 pour préparer une composition nutritionnelle et/ou pharmaceutique, **caractérisée en ce que** ladite bactérie et les peptides SEQ.ID.NO : 1-6 sont présents dans une quantité qui est suffisante pour avoir un effet probiotique sur ladite composition nutritionnelle et/ou pharmaceutique dont un individu a besoin, de préférence contre *Helicobacter pylori.*

12. Composition nutritionnelle, comprenant une quantité efficace de la souche *Bifidobacterium bifidum* CECT 7366 selon les revendications 1-3 ou la souche *Bifidobacterium bifidum* CECT 7366 selon les revendications 1-3 et les peptides avec acide aminé SEQ.ID.NO: 1 à 6, conjointement avec des quantités appropriées d'autres aliments.

13. Composition nutritionnelle selon la revendication 12, dans laquelle les autres aliments sont choisis dans le groupe comprenant de la crème glacée, des céréales, des jus, des barres énergétiques, des produits laitiers tels que du lait entier, du lait écrémé, du lait fermenté, du lait en poudre, du fromage, des yaourts ou d'autres produits laitiers.

14. Composition nutritionnelle selon la revendication 13, **caractérisée en ce que** c'est un yaourt.

15. Composition de yaourt selon les revendications 14, **caractérisée en ce que** les cellules de la souche *Bifidobacterium bifidum* CECT 7366 selon les revendications 1-3 ont une viabilité d'au moins 80 % pour une période de 15 jours lorsqu'elles sont maintenues à 5 °C.

16. Composition nutritionnelle selon les revendications 12-15, **caractérisée en ce que** la souche *Bifidobacterium bifidum* CECT 7366 selon les revendications 1-3 est présente dans une quantité entre 10⁵-10¹¹ ufc/g de la composition.

17. Souche de *Bifidobacterium bifidum* CECT 7366 selon les revendications 1-3 pour une utilisation dans la prévention ou la guérison des ulcères gastriques ou du cancer de l'estomac causés par infection *Helicobacter pylori.*

18. Composition pharmaceutique comprenant, un lyophilisat selon les revendications 9 à 10 dans une quantité physiologiquement efficace, conjointement avec les véhicules et/ou adjuvants nécessaires pharmaceutiquement acceptables pour la prévention ou la guérison des ulcères gastriques ou du cancer de l'estomac causés par infection *Helicobacter pylori.*

19. Composition pharmaceutique selon les revendications 18, **caractérisée en ce que** ladite composition est sous forme solide, telle que, par exemple, des pilules, des comprimés, etc. ; sous forme liquide, en suspension ou dispersion.

20. Composition pharmaceutique selon les revendications 18-19, **caractérisée en ce qu'**elle comprend en outre des antibiotiques pour le traitement des ulcères gastriques.

21. Composition pharmaceutique selon les revendications 18-20, **caractérisée en ce que** la souche est présente dans une quantité entre 10⁷-10¹¹ ufc/g.
